# EUROPEAN PATENT APPLICATION

(11) **EP 4 471 141 A1**
(43) Date of publication of application: **04.12.2024**
(21) Application number: 21969695.2
(22) Date of filing: 31.12.2021
(51) Int. Cl.: C12N 15/113, C40B 40/06, C12Q 1/6869, C12Q 1/6806

(54) **NUCLEIC ACID MOLECULE CAPABLE OF BLOCKING MOTOR PROTEIN, AND CONSTRUCTION METHOD AND APPLICATION THEREOF**

(71) Applicant: BGI Shenzhen, Shenzhen, Guangdong 518083 (CN)
(72) Inventor: WANG, Ou, Shenzhen, Guangdong 518083 (CN); SHI, Xiao, Shenzhen, Guangdong 518083 (CN); XU, Xun, Shenzhen, Guangdong 518083 (CN); ZHANG, Wenwei, Shenzhen, Guangdong 518083 (CN); GUO, Fei, Shenzhen, Guangdong 518083 (CN); BAO, Siyu, Shenzhen, Guangdong 518083 (CN); DONG, Yuliang, Shenzhen, Guangdong 518083 (CN); ZENG, Tao, Shenzhen, Guangdong 518083 (CN)
(74) Representative: Burger, Hannes Alfred
(86) International application number: PCT/CN2021/143658
(87) International publication number: WO 2023/123344

(57) **Abstract**

The present invention relates to a nucleic acid molecule capable of blocking motor protein, a library containing the nucleic acid molecule, a method for constructing the nucleic acid molecule or library containing the same, and an application of the nucleic acid molecule or library containing same. The nucleic acid molecule contains at the end a modified nucleotide capable of blocking a motor protein, and the modified nucleotide is as shown in Formula I or Formula I':

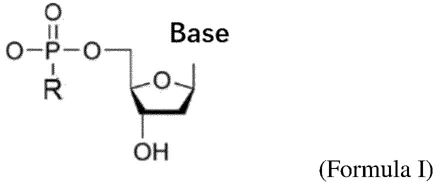

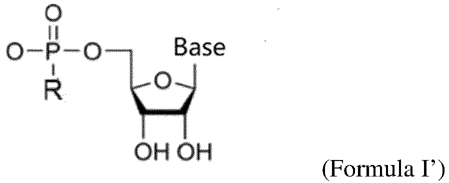

## Description

### Technical Field

The present invention generally relates to the technical field of nucleic acid sequencing. In particular, the present invention relates to a nucleic acid molecule capable of blocking a motor protein, a library containing the same, a method of constructing the nucleic acid molecule or the library containing the same, and uses of the nucleic acid molecule or library containing the same.

### Background

Nucleic acid sequencing has become an indispensable and important research method in the field of life science research. Along with this, genomics technology based on large-scale sequencing data has also played an important role in different research and application directions, such as tracing the origin of complex diseases and dynamic monitoring of their development processes, directional breeding of economic crops and animals, research and protection of different biological genetic resources, etc.

However, the prerequisite for the above research is how to use high-throughput parallel sequencing to obtain high-precision and complete nucleic acid sequences. At present, the mainstream sequencing-by-synthesis technology has high sequencing accuracy, and the original accuracy rate of most sequenced bases can reach more than 99.9%, but the read length thereof is short, and it is difficult to restore the complete target nucleic acid sequence using these shortread length sequences, whether via resequencing alignment or de novo assembly.

Therefore, how to obtain long-range information in nucleic acid sequences has become one of the hot issues in the current sequencing technology research. Commercial companies that can currently provide long-read sequencing solutions mainly include: Pacific Bioscience (abbreviated as PacBio, the same below) in the United States, which provides single-molecule real-time sequencing, and Oxford Nanopore Technology (abbreviated as ONT, the same below) in the United Kingdom, which provides nanopore sequencing. In addition, companies such as Quantum SI and Genia in the United States, and Qitan Tech, Geneus, and Axbio in China have all entered this technology field and released prototypes.

Existing nanopore sequencing technology requires the use of motor proteins to drive the unwinding of double-stranded DNA to form single-stranded DNA, which then enters the nanopore, and base sequences are distinguished according to current changes caused by different bases. In current technology, the role of motor proteins is very critical. It undertakes two main functions. One is to unwind double-stranded DNA and ensure that single strands of DNA enter the pore, thereby ensuring the uniqueness of sequence recognition; the other is to control the speed of DNA passing through the nanopore so that it is not too fast or too slow, thereby ensuring that the base recognition algorithm can distinguish different sequences very well. However, it needs to be emphasized that in its natural state, the motor protein can spontaneously unwind double-stranded DNA once it is combined with the library adapter in the presence of ATP in the system. This will cause the library to be sequenced to totally become single strands, and cannot be sequenced normally. In order to solve this problem, the existing technical solution is to introduce a compound such as short-chain polyethylene glycol (e.g., iSp18) or polypropylene glycol (e.g., SpC3) into an oligonucleotide sequence that acts as an adapter in the library to be sequenced, which reduces the negative charge on this segment of the DNA chain and inhibits the motor protein from moving forward further.

However, there are some problems with the current nanopore sequencing technology solution. During sequencing, the motor protein will first stay at the spacer, then when the library moves to the nanopore, the spacer will pass through the active area of the motor protein under the action of electric field force, and lose its blocking effect. The motor protein then begins to unwind normally, thereby initiating sequencing. This means that the existing technical means inevitably need to detect an adapter sequence after the spacer during sequencing, and this sequence may cause crosstalk to subsequent information analysis results. Since a fixed adapter sequence is first detected at the beginning of sequencing, an additional process of filtering adapter sequence is required in subsequent biological information analysis, resulting in a waste of part of the sequencing read length; and due to the low accuracy of nanopore sequencing, the adapter sequence may be processed incompletely, which ultimately produces crosstalk in subsequent analyses; in addition, experimental observations indicate that special adapter sequences formed by short-chain polyethylene glycol (e.g., iSp18) or polypropylene glycol (e.g., SpC3) have weaker signal characteristics, so that it is difficult for downstream algorithms to determine the starting point of sequencing.

With the advancement of science and technology, the current clinical sample mutation detection is no longer satisfied with detecting only small-scale variations (single nucleotide mutations and small deletion/insertion mutations), and some genetic abnormalities caused by large-scale structural variations are gradually being analyzed. Since structural variation detection is more sensitive to sequencing read length, long-read sequencing technology will gradually shift from the field of scientific and technological services to the field of clinical detection, and the requirements for accuracy and cost control of sequencing technology will also increase.

In terms of accuracy, the Hi-Fi sequencing method based on circular consensus sequencing launched by PacBio in 2019 can achieve an average sequencing accuracy rate of more than 99% by repeating 5 reads. However, since it is required to repeatedly sequence the same molecule for several times, the cost is high.

In terms of cost, the latest PromethION 48 system launched by Oxford Nanopore (ONT) can achieve single Gb sequencing costs between US$2 to US$16, which is gradually approaching the cost of synthetic sequencing methods that are widely used in the market. Although the accuracy rate of this system is still insufficient as compared to the PacBio's circular consensus sequencing method, according to the latest data disclosed by Oxford Nanopore, its accuracy rate can reach 98.4%, which has been significantly improved as compared to the early nanopore data.

Although the current long-read sequencing technology still has some shortcomings, it represents an important direction for future technology development. With the advancement of technology, long-read sequencing is expected to penetrate into the field of clinical detection. The nanopore sequencing solution has become a key breakthrough direction in research due to its low cost and still having room for further improvement in accuracy.

Different from conventional sequencing-by-synthesis, the library for nanopore sequencing needs to be combined with motor proteins (usually helicases) in advance, and the motor proteins need to be kept at the adapter position of the library to be sequenced without unwinding the chain to be sequenced before sequencing formally starts. This requires the introduction of motor protein spacer structures during the construction of the library to be sequenced so as to achieve the above purpose. There remains a need in the art for efficient motor protein spacer structures.

### Contents of the Invention

The present application relates to an innovative study on efficient motor protein spacer structures.

For one object of the present invention, a target nucleic acid molecule to be sequenced comprises at its end a modified nucleotide capable of blocking a motor protein, thereby achieving the blocking effect against the motor protein and obtaining better single-molecule sequencing data.

For another object of the present invention, an end of a target nucleic acid molecule is modified during the end phosphorylation or end-repair process of the target nucleic acid molecule, thereby obtaining a nucleic acid molecule comprising at its end a modified nucleotide capable of blocking a motor protein.

For another object of the present invention, the detection of an adapter sequence after a spacer is avoided during sequencing, thereby further simplifying the biological information analysis process.

For further another object of the present invention, the motor protein blocking efficiency is effectively improved, thereby enhancing the adapter recognition signal.

In a first aspect, the present invention provides a nucleic acid molecule capable of blocking a motor protein, the nucleic acid molecule comprising at its end a modified nucleotide capable of blocking the motor protein.

In one embodiment, the nucleic acid molecule is DNA or RNA.

In one embodiment, the nucleic acid molecule comprises at its 5' end a modified nucleotide capable of blocking a motor protein. Preferably, the nucleotide at the 5' end of the nucleic acid molecule is phosphorylated by a methylphosphate group to form a modified nucleotide capable of blocking a motor protein.

In one embodiment, the nucleic acid molecule comprises at its 3' end one or more modified nucleotides capable of blocking motor proteins.

In one embodiment, the nucleic acid molecule comprises at both its 5' end and 3' end modified nucleotides capable of blocking motor proteins.

In one embodiment, the modified nucleotide capable of blocking a motor protein refers to a nucleotide bearing a group capable of blocking a motor protein, and such group capable of blocking a motor protein may be selected from, but not limited to: alkyl, fluorophore, streptavidin and/or biotin, cholesterol, methylene blue, dinitrophenol (DNP), digoxigenin ligand and/or anti-digoxigenin ligand, dibenzocyclooctynyl, etc. In a preferred embodiment, such group capable of blocking a motor protein may be alkyl, for example, but not limited to, methyl, ethyl, propyl, isopropyl, silylmethyl or boranyl. Preferably, alkyl can replace a negatively charged oxygen atom on the phosphate group in the nucleotide, and other groups capable of blocking a motor protein can be connected to the base, phosphate, ribose or deoxyribose.

In one embodiment, the α (alpha)-phosphate of the modified nucleotide located at the end of the nucleic acid molecule is modified. Preferably, one negatively charged oxygen atom in the α-phosphate is substituted by the above-mentioned group capable of blocking a motor protein. In a more preferred embodiment, one negatively charged oxygen atom in the α-phosphate is substituted by alkyl, more preferably by methyl, ethyl, propyl, isopropyl, silylmethyl or boranyl.

In a preferred embodiment, the modified nucleotide can be selected from, but not limited to: ribonucleotides or deoxyribonucleotides with alkyl (e.g., methyl, ethyl, propyl, isopropyl, silylmethyl or boranyl) substitution on the α-phosphate; ribonucleotides or deoxyribonucleotides with nucleoside modification, for example, 3-methyladenine nucleotide, 7-methylguanosine nucleotide, 1, N⁶-ethenoadenine nucleotide, hypoxanthine nucleotide, uracil nucleotide, etc.; or ribonucleotides or deoxyribonucleotides with modification at the sugar ring, for example, locked nucleotides, peptide nucleotides or threose nucleotides, etc.

In one embodiment, the modified nucleotide contained at the end of the nucleic acid molecule can be represented by Formula I: 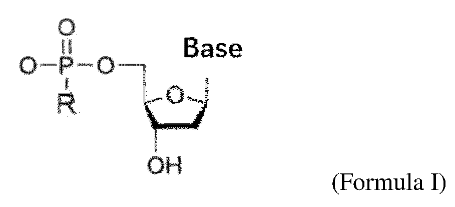
wherein, Base represents any base (i.e., A, T, G, C or U or other bases),
one negatively charged oxygen atom in α-phosphate is replaced by an R group, in which the R group may be selected from, but is not limited to: alkyl, fluorophore, streptavidin and/or biotin, cholesterol, methylene blue, dinitrophenol (DNP), digoxigenin ligand and/or anti-digoxigenin ligand and dibenzocyclooctynyl; preferably, the R group may be alkyl, for example, but not limited to: methyl, ethyl, propyl, isopropyl, silylmethyl or boranyl; more preferably, the R group is methyl.

In a preferred embodiment, the modified nucleotide contained at the end of the nucleic acid molecule can be represented by Formula II: 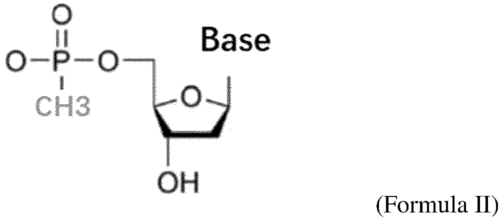 wherein, Base represents any base.

In other words, in the nucleic acid molecule, one negatively charged oxygen atom in the α-phosphate of the modified nucleotide (Formula II) contained at its end is substituted by methyl.

In a preferred embodiment, the modified nucleotide contained at the end of the nucleic acid molecule may be adenosine methylphosphate, as shown in Formula III:

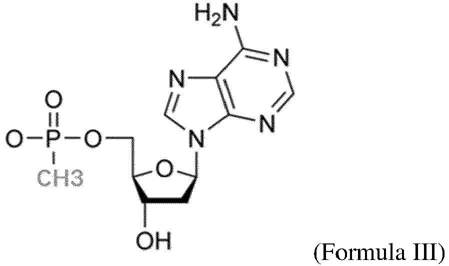

It is easy for those skilled in the art to understand that although Formula I, Formula II and Formula III show deoxyribose monophosphate nucleosides, ribose monophosphate nucleosides with similar structures are also encompassed in the scope of the present invention. For example, when the nucleic acid molecule is RNA, the modified nucleotide contained at its end is correspondingly a ribose monophosphate nucleoside in which one negatively charged oxygen atom in the α-phosphate is replaced by an R group. The corresponding structures are shown in Formula I', Formula II', and Formula III', respectively:

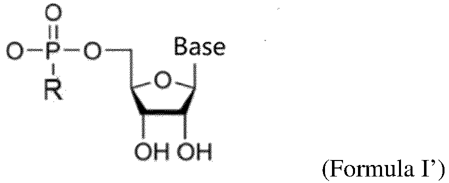

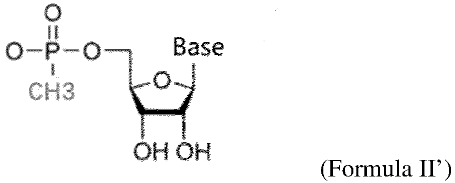

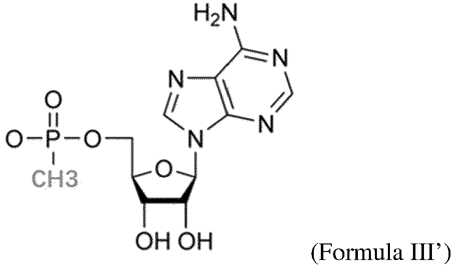

In Formula I', Formula II' and Formula III', the definitions of Base and R are the same as those in Formula I, Formula II and Formula III, respectively.

In one embodiment, the nucleic acid molecule containing at its end a modified nucleotide capable of blocking a motor protein is used for single-molecule sequencing, for example, for nanopore sequencing.

In one embodiment, the nucleic acid molecule containing at its end a modified nucleotide capable of blocking a motor protein constitutes a library, for example, a sequencing library. This sequencing library can be used for single-molecule sequencing, for example, for nanopore sequencing.

In one embodiment, when a nucleic acid molecule containing at its 5' end a modified nucleotide capable of blocking a motor protein is used for single-molecule sequencing, for example, for nanopore sequencing, a motor protein with movement direction from 5' to 3', for example, but not limited to, a T4 bacteriophage DNA helicase Dda and mutant thereof (hereinafter referred to as Dda), *Deinococcus radiodurans* DNA helicase RecD2 (hereinafter referred to as RecD2), or human DNA helicase Pif1 (hereinafter referred to as Pif1), is used.

In one embodiment, when a nucleic acid molecule containing at its 3' end a modified nucleotide capable of blocking a motor protein is used for single-molecule sequencing, for example, for nanopore sequencing, a motor protein with movement direction from 3' to 5' direction, for example, but not limited to, *Escherichia coli* DNA helicase Rep and its mutant Rep-X, *Drosophila* DNA helicase RecQ4 (hereinafter referred to as RecQ4), or *Methanothermobacter thermautotrophicus* DNA helicase Hel308 (hereinafter referred to as Hel308) is used.

In one embodiment, when a nucleic acid molecule respectively containing at its 5' end and 3' end modified nucleotides capable of blocking a motor protein is used for single-molecule sequencing, for example, for nanopore sequencing, a motor protein with movement direction from 5' to 3' can be used, and a motor protein with movement direction from 3' to 5' can also be used. Those skilled in the art can select appropriate motor proteins based on actual needs.

Those skilled in the art should understand that examples of motor proteins are known in the art, and those skilled in the art can select appropriate motor proteins according to actual needs.

In other embodiments, the present invention provides a sequencing library, the sequencing library comprises at least one nucleic acid molecule capable of blocking a motor protein, wherein the nucleic acid molecule comprises at its end a modified nucleotide capable of blocking a motor protein. Wherein, the "nucleic acid molecule capable of blocking a motor protein" and "modified nucleotide" are as defined above.

In a second aspect, the present invention relates to a method for preparing the nucleic acid molecule capable of blocking a motor protein according to the first aspect, the method comprising a step of making the target nucleic acid molecule contain at its end a modified nucleotide capable of blocking a motor protein. The nucleic acid molecule may be DNA or RNA.

In one embodiment, the modified nucleotides capable of blocking a motor protein refer to a nucleotide bearing a group capable of blocking a motor protein. Such group capable of blocking a motor protein may be selected from, but not limited to: alkyl, fluorophore, streptavidin and/or biotin, cholesterol, methylene blue, dinitrophenol (DNP), digoxigenin ligand and/or anti-digoxigenin ligand, dibenzocyclooctynyl, etc. In a preferred embodiment, such group capable of blocking a motor protein may be alkyl, for example, but not limited to, methyl, ethyl, propyl, isopropyl, silylmethyl or boranyl.

In one embodiment, the α (alpha)-phosphate of the modified nucleotide located at the end of the nucleic acid molecule is modified. Preferably, one negatively charged oxygen atom in the α-phosphate is substituted by the above-mentioned group capable of blocking a motor protein. In a more preferred embodiment, one negatively charged oxygen atom in the α-phosphate is substituted by alkyl, more preferably by methyl, ethyl, propyl, isopropyl, silylmethyl or boranyl.

In a preferred embodiment, the modified nucleotide may be selected from, but not limited to: ribonucleotides or deoxyribonucleotides with alkyl (e.g., methyl, ethyl, propyl, isopropyl, silylmethyl or boranyl) substitution on the α-phosphate; ribonucleotides or deoxyribonucleotides with nucleoside modification, for example, 3-methyladenine nucleotide, 7-methylguanosine nucleotide, 1, N⁶-ethenoadenine nucleotide, hypoxanthine nucleotide, uracil nucleotide, etc.; or ribonucleotides or deoxyribonucleotides in which the sugar ring is modified, for example, locked nucleotides, peptide nucleotides or threose nucleotides, etc.

In one embodiment, the modified nucleotide contained at the end of the nucleic acid molecule can be represented by Formula I, preferably Formula II, more preferably Formula III, wherein Formula I, Formula II and Formula III are all as defined in the first aspect; or can be represented by Formula I', preferably Formula II', more preferably Formula III', wherein Formula I', Formula II' and Formula III' are all as defined in the first aspect.

In one embodiment, the method of generating the nucleic acid molecule capable of blocking a motor protein comprises: introducing a phosphate group bearing a group capable of blocking a motor protein into the 5'-end nucleotide of the target nucleic acid molecule through 5'-end phosphorylation, wherein the group capable of blocking a motor protein may be selected from, but not limited to: alkyl, fluorophore, streptavidin and/or biotin, cholesterol, methylene blue, dinitrophenol (DNP), digoxigenin ligand and/or anti-digoxigenin ligand, dibenzocyclooctynyl, etc. In a preferred embodiment, such group capable of blocking a motor protein may be alkyl, for example, but not limited to, methyl, ethyl, propyl, isopropyl, silylmethyl or boranyl.

In a preferred embodiment, in the 5'-end phosphorylation reaction, the target nucleic acid molecule is reacted with a modified NTP represented by Formula IV (as a reaction substrate), so that the phosphate group with R group at the γ-position of the modified NTP represented by Formula IV is transferred to the 5'-end nucleotide of the target nucleic acid molecule, 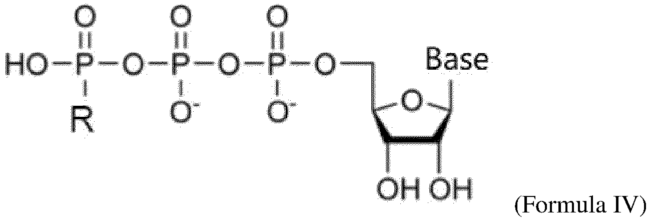 in Formula IV, Base represents any base, and one negatively charged oxygen atom of the phosphate group at the γ-position is substituted by R group, wherein the R group represents a non-polar group or a weakly polar group, which can be selected from, but not limited to, methyl, ethyl, propyl, isopropyl, silylmethyl or boranyl.

In a more preferred embodiment, R is methyl. In the most preferred embodiment, R is methyl and Base is adenine (A), that is, the modified NTP represented by Formula IV is γ-methylphosphate ATP represented by Formula V:

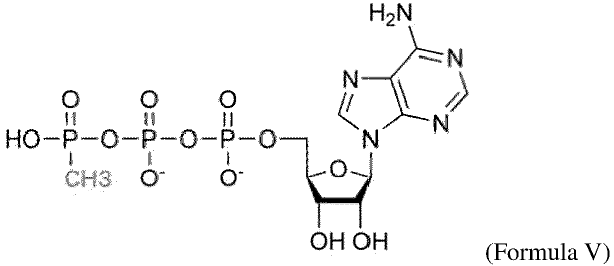

Those skilled in the art can easily understand that although Formula IV and Formula V represent ribose nucleoside triphosphates, deoxyribose nucleoside triphosphates with similar structures are also within the scope of the present invention. The corresponding structures are represented by Formula IV' and Formula V', respectively: 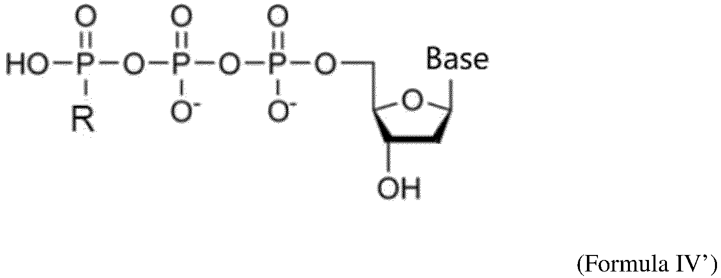 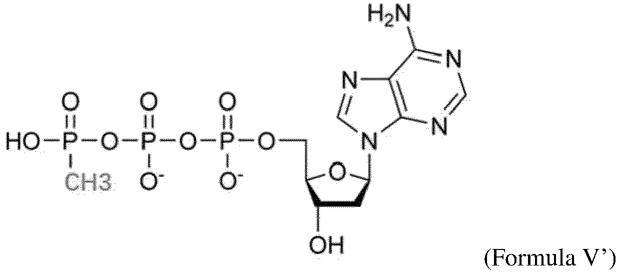 in Formula IV' and Formula V', the definitions of Base and R are the same as those in Formula IV and Formula V, respectively.

In a preferred embodiment, 5'-end phosphorylation occurs in the presence of a polynucleotide kinase (PNK).

In a more preferred embodiment, the polynucleotide kinase (PNK) can be selected from, but is not limited to: nucleic acid kinases, phosphotransferases or phosphatases. For example, the polynucleotide kinase may be a T4 phage-derived polynucleotide kinase, a human-derived polynucleotide kinase 3'-phosphatase, a human-derived polynucleotide 5'-hydroxykinase NOL9, or a human-derived polynucleotide 5'-hydroxykinase Clp 1.

In one embodiment, the reaction substrate of the 5'-end phosphorylation reaction is preferably represented by Formula IV, more preferably Formula V; or may be preferably represented by Formula IV', more preferably Formula V'.

In one embodiment, the method for generating the nucleic acid molecule capable of blocking a motor protein comprises: introducing a modified nucleotide into the 3' end of the target nucleic acid molecule through a polymerase in a step of performing end-repair of the target nucleic acid molecule and/or a step of adding A to the 3' end of the target nucleic acid molecule.

Those skilled in the art should understand that the "step of performing end-repair" can repair the 5' end and 3' end of the nucleic acid molecule, and can also repair the nick position in the nucleic acid molecule; the "step of adding A at 3' end" is a routine operation in this field, which is not limited to introducing dAMP at the 3' end of the nucleic acid molecule, but can also introduce dNMP (N=C, G or T) that is different from dAMP, and can change the corresponding bases (G, C, A) on the adapter without affecting the effect.

Preferably, in the step of performing end-repair of the target nucleic acid molecule and/or the step of adding A to the 3' end of the target nucleic acid molecule, the target nucleic acid molecule is reacted with the modified dNTP represented by Formula VI through a polymerase, thereby introducing the modified dNMP into the 3' end of the target nucleic acid molecule, in which the phosphorus atom of the modified dNMP is connected with the R group capable of blocking a motor protein (also known as R group-modified adenosine phosphate): 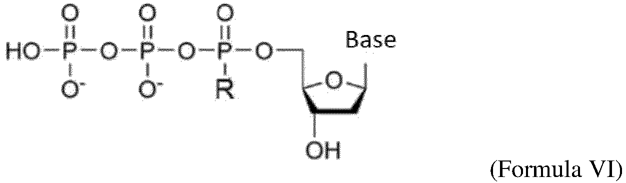 in Formula VI, Base represents any base, and the negatively charged oxygen atom of the phosphate group at the α-position is substituted by R group, wherein the R group represents a non-polar group or a weakly polar group, which can be selected from, but not limited to, methyl, ethyl, propyl, isopropyl, silylmethyl or boranyl.

In a preferred embodiment, the modified dNTP is dATP in which one negatively charged oxygen atom of the α-phosphate group is substituted by methyl. That is, the modified dNTP is α-methyl phosphate dATP represented by the following Formula VII:

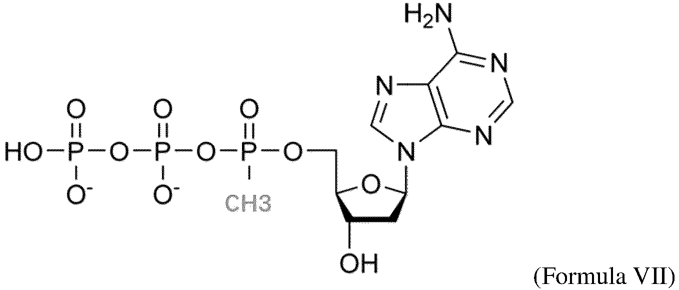

In one embodiment, during the step of performing end-repair of the target nucleic acid molecule, one or more modified nucleotides can be transferred to the 3' end of the target nucleic acid sequence by a polymerase. Preferably, in the step of performing end-repair, one or more modified nucleotides can be introduced into the 3' end of the target nucleic acid sequence in a template-dependent manner. Alternatively, multiple modified nucleotides can also be introduced into the 3' end of the target nucleic acid sequence in a template-independent manner by a terminal transferase.

In one embodiment, during the step of adding A to the 3' end of the target nucleic acid molecule, one modified nucleotide can be transferred to the 3' end of the target nucleic acid sequence by a polymerase.

The polymerase may be, but is not limited to: nucleic acid polymerases, transcriptases, reverse transcriptases or terminal transferases. For example, the polymerase may be *Thermococcus gorgonarius* (Tgo) DNA polymerase or mutant thereof (e.g., PGV2), *Thermus aquaticus* (Taq) polymerase I, or *Escherichia coli* polymerase I Klenow fragment, but is not limited thereto. Examples of polymerases are known in the art, and those skilled in the art can select a suitable polymerase according to actual needs.

In one embodiment, during the step of performing end-repair and/or the step of adding A to the 3' end, multiple modified nucleotides can be transferred to the 3' end of the target nucleic acid sequence using a polymerase, thereby achieving better blocking effect against the motor protein.

Alternatively, the nucleic acid molecule capable of blocking a motor protein can also be generated by chemical synthesis. For example, for a short target nucleic acid molecule, chemical synthesis can be used to generate a nucleic acid molecule containing at its end (for example, at its 5' end, at its 3' end, or at both its 3' and 5' ends) one or more modified nucleotides. The modified nucleotide may be represented by Formula I, preferably Formula II, more preferably Formula III, wherein Formula I, Formula II and Formula III are all as defined in the first aspect.

In a third aspect, the present invention provides a method for constructing a sequencing library capable of blocking motor proteins, the sequencing library comprising at least one nucleic acid molecule capable of blocking motor proteins according to the first aspect, wherein the method comprises a step of making a target nucleic acid molecule contain at its end (i.e., its 5' end or 3' end, or both its 5' and 3' ends) a modified nucleotide capable of blocking a motor protein during the construction of the sequencing library. The nucleic acid molecule may be DNA or RNA.

Those skilled in the art can understand that the method of generating the nucleic acid molecule capable of blocking a motor protein as described in the second aspect of the present invention can be used in the method for constructing a sequencing library, thereby obtaining the sequencing library comprising a nucleic acid molecule capable of blocking a motor protein. The sequencing library can be used for single-molecule sequencing, for example, for nanopore sequencing.

In one embodiment, when the nucleic acid molecule in the sequencing library comprises at its 5' end a modified nucleotide capable of blocking a motor protein and the sequencing library is used for single-molecule sequencing, for example, for nanopore sequencing, a motor protein with movement direction from 5' to 3' is used, which is, for example, but not limited to, Dda or its mutant, recD2, or Pif1.

In one embodiment, when the nucleic acid molecule in the sequencing library comprises at its 3' end a modified nucleotide capable of blocking a motor protein and the sequencing library is used for single-molecule sequencing, for example, for nanopore sequencing, a motor protein with movement direction from 3' to 5' is used, which is, for example, but not limited to, Rep or its mutant Rep-X, RecQ4, or Hel308.

Those skilled in the art should understand that examples of motor proteins are known in the art, and those skilled in the art can select appropriate motor proteins according to actual needs.

In a fourth aspect, the present invention relates to a use of the nucleic acid molecule capable of blocking a motor protein of the first aspect or the library comprising the same. The nucleic acid molecule capable of blocking a motor protein or the library comprising the same can be used in the field of single-molecule sequencing. For example, single-molecule nucleic acid sequence sequencing can be performed by incubating the nucleic acid molecule or the library containing the same with motor proteins, and adding to an electrophysiological detection system containing nanopore proteins.

The nucleic acid molecule capable of blocking a motor protein or the library containing the same can also be used to detect motor protein activity. For example, by incubating the nucleic acid molecule capable of blocking a motor protein or the library containing the same with the motor protein, the nucleic acid molecule capable of blocking the motor protein can be used as a negative control for the identification of motor protein activity since the motor protein is incapable to unwind the nucleic acid molecule capable of blocking the motor protein.

In a fifth aspect, the present invention relates to a single-molecule sequencing method, preferably a nanopore sequencing method, in which the method is performed by using the nucleic acid molecule capable of blocking a motor protein of the first aspect of the present invention or the library comprising the same.

In one embodiment, the single-molecule sequencing method comprises:
(1) making a target nucleic acid molecule contain at its end a modified nucleotide capable of blocking a motor protein, so as to obtain a sequencing nucleic acid molecule or sequencing library, which can be achieved by the method described in the second aspect or the third aspect;
(2) ligating the sequencing nucleic acid molecule or sequencing library to a sequencing adapter and then incubating with a motor protein, or incubating the sequencing adapter with the motor protein and then ligating to the sequencing nucleic acid molecule or sequencing library;
(3) adding the product of step (2) to an electrophysiological detection system containing nanopores to perform single-molecule nanopore sequencing.

For the motor protein and/or sequencing adapter, as well as suitable single-molecule sequencing devices used in the single-molecule sequencing method, those skilled in the art can make appropriate selections based on actual needs.

In one embodiment, the single-molecule sequencing method comprises:
(1) after performing end-repair of a target nucleic acid molecule, transferring a phosphate group with a group capable of blocking a motor protein to the 5'-end nucleotide of the target nucleic acid molecule by using the modified nucleotide represented by Formula IV or Formula V as a substrate under the action of a polynucleotide kinase, and then performing an end-adding A reaction;
(2) ligating the nucleic acid molecule, into which the phosphate group with the group capable of blocking a motor protein is introduced at the 5' end, to a sequencing adapter under the action of a ligase, and then incubating with a motor protein with movement direction from 5' to 3'; alternatively, firstly incubating the sequencing adapter with the motor protein with movement direction from 5' to 3', and then ligating to the nucleic acid molecule into which the phosphate group with the group capable of blocking a motor protein is introduced at the 5' end, in which the order of the two does not affect process effect;
(3) adding the above ligation product to an electrophysiological detection system containing nanopores to perform single-molecule nanopore sequencing.

In one embodiment, the single-molecule sequencing method comprises:
(1) using a conventional nucleotide to perform end-repair and phosphorylation of a target nucleic acid molecule under the action of a polymerase, and then using a modified nucleotide represented by Formula VI or Formula VII as a substrate to perform an end-adding A reaction, thereby introducing one modified nucleotide at the 3' end of the target nucleic acid molecule;
(2) ligating the nucleic acid molecule containing at its 3' end the modified nucleotide to a sequencing adapter under the action of a ligase, and then incubating with a motor protein with movement direction from 3' to 5'; alternatively, incubating firstly the sequencing adapter with the motor protein with movement direction from 3' to 5', and then ligating to the nucleic acid molecule containing at its 3' end the modified nucleotide, in which the order of the two does not affect process effect;
(3) adding the above ligation product to an electrophysiological detection system containing nanopores to perform single-molecule nanopore sequencing.

In another embodiment, the single-molecule sequencing method comprises:
(1) using a conventional nucleotide to perform end-repair and phosphorylation reactions on a target nucleic acid molecule under the action of a polymerase, and then using a modified nucleotide represented by Formula VI or Formula VII as a substrate to perform an end-adding A reaction, thereby introducing one modified nucleotide into the 3' end of the target nucleic acid molecule;
(2) ligating the nucleic acid molecule containing at its 3' end the modified nucleotide to a sequencing adapter under the action of a ligase, and then incubating with a motor protein with movement direction from 3' to 5'; alternatively, incubating first the sequencing adapter with the motor protein with movement direction from 3' to 5', and then ligating to the nucleic acid molecule containing at its 3' end the modified nucleotide, in which the order of the two does not affect process effect;
(3) adding the above ligation product to an electrophysiological detection system containing nanopores to perform single-molecule nanopore sequencing.

In another embodiment, the single-molecule sequencing method comprises:
(1) using a modified nucleotide represented by Formula VI or Formula VII as a substrate to perform end-repair and end-adding A reaction on a target nucleic acid molecule under the action of a polymerase, thereby introducing one or more modified nucleotides to the 3' end of the target nucleic acid molecule;
(2) then using a modified nucleotide represented by Formula IV or Formula V as a substrate to perform a phosphorylation reaction, thereby introducing a phosphate group with a group capable of blocking a motor protein into the 5'-end nucleotide of the target nucleic acid molecule;
(3) ligating the nucleic acid molecule containing modified nucleotides at both the 3' end and the 5' end to a sequencing adapter under the action of a ligase, and then incubating with a motor protein with movement direction from 3' to 5'; alternatively, incubating first the sequencing adapter with the motor protein with movement direction from 3' to 5', and then ligating to the nucleic acid molecule containing modified nucleotides at both the 3' end and the 5' end, in which the order of the two does not affect the process effect;
(4) adding the above ligation product to an electrophysiological detection system containing nanopores to perform single-molecule nanopore sequencing.

Those skilled in the art will understand that when the 5' end and 3' end of the target nucleic acid molecule respectively contain modified nucleotides capable of blocking motor proteins, in single-molecule sequencing (e.g., nanopore sequencing) a motor protein that moves in the 5' to 3' direction can be used, and a motor protein that moves in the 3' to 5' direction can also be used. Those skilled in the art can select appropriate motor proteins based on actual needs.

### Advantages of the present invention

Compared with a sequencing sequence or sequencing library that does not contain at its end the modified nucleotide, the nucleic acid molecular or sequencing library of the present invention that contains at its end the modified nucleotide capable of blocking a motor protein can avoid detecting an adapter sequence after a spacer during sequencing, which simplifies the biological information analysis and processing process, reduces the crosstalk of adapter sequences to subsequent analysis, and enhances the signal intensity of special sequences, thereby providing help for base identification tools in better determining the starting point of a sequencing sequence.

### Brief Description of the Drawings

The features and advantages of the present invention will become more apparent by referring to the accompanying drawings in conjunction with the disclosure of the present application.

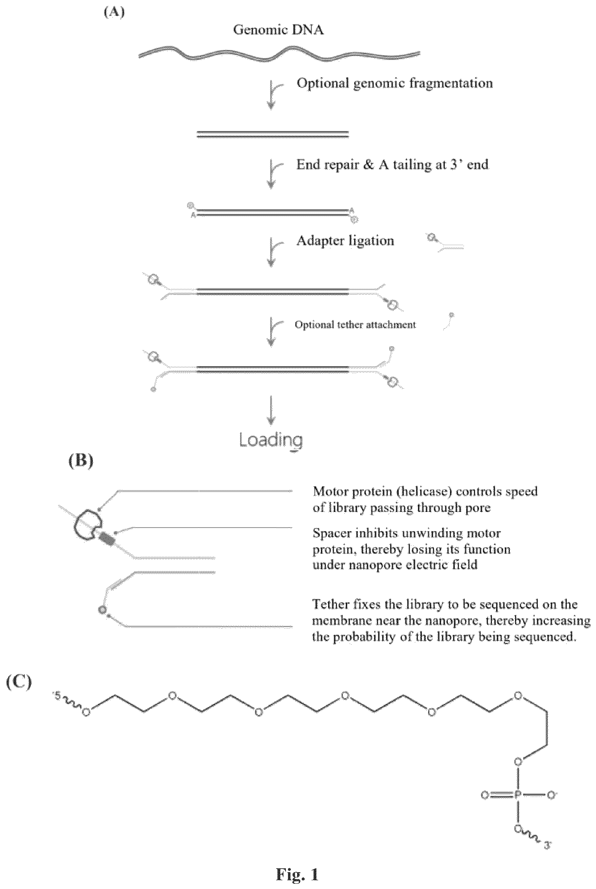

Fig. 1 shows the existing construction scheme of a nanopore sequencing library in the field, wherein (A) shows Oxford Nanopore's library construction flow chart for nanopore sequencing, (B) shows a structural schematic diagram of an adapter for ligating a sequencing library to a tether sequence, and (C) shows a structural formula (i.e., iSp18 monomer structural formula) of an exemplary spacer sequence.

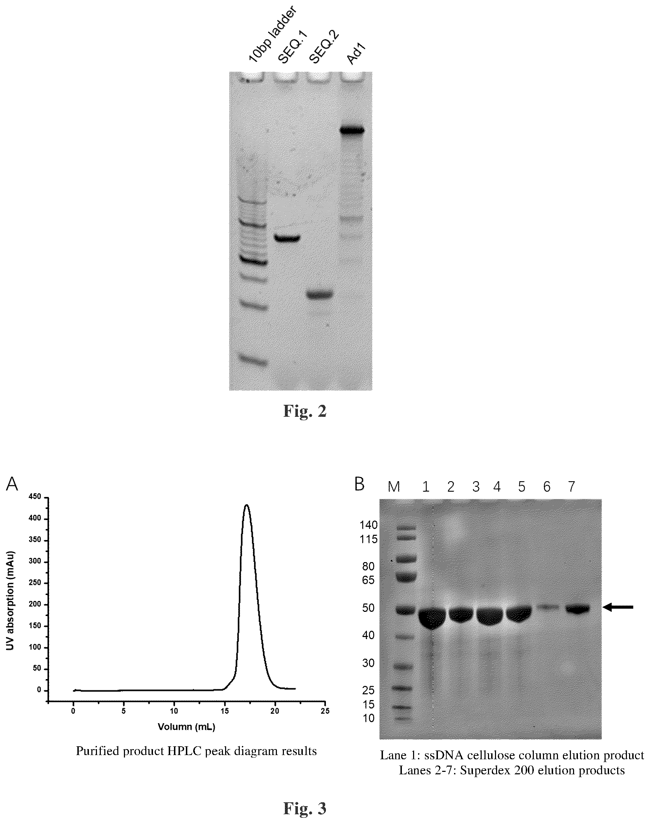

Fig. 2 shows the results of non-denaturing polyacrylamide gel electrophoresis of an adapter annealing product, wherein the first lane from the left is the 10bp DNA Ladder, the second lane from the left is the SEQ ID NO.1 sequence, the third lane from the left is the SEQ ID NO.2 sequence, and the fourth lane from the left is the adapter product Ad1 after annealing of SEQ ID NO.1 and SEQ ID NO.2 sequences.
Fig. 3 shows the HPLC and SDS-PAGE electrophoresis purity results of the Dda motor protein (SEQ ID NO.4) after the expression and purification, wherein Fig. 3A shows the HPLC peak chart, and the HPLC results show that the purity of the protein product is above 90%; Fig. 3B shows the SDS-PAGE electrophoresis image, and the results show that as the purification step proceeded, the protein purity continued to improve, in which M represents the molecular weight gradient, Lane 1 is the ssDNA cellulose column elution product, lanes 2 to 7 are the Superdex 200 column elution products, and the arrow points to the Dda motor protein band.

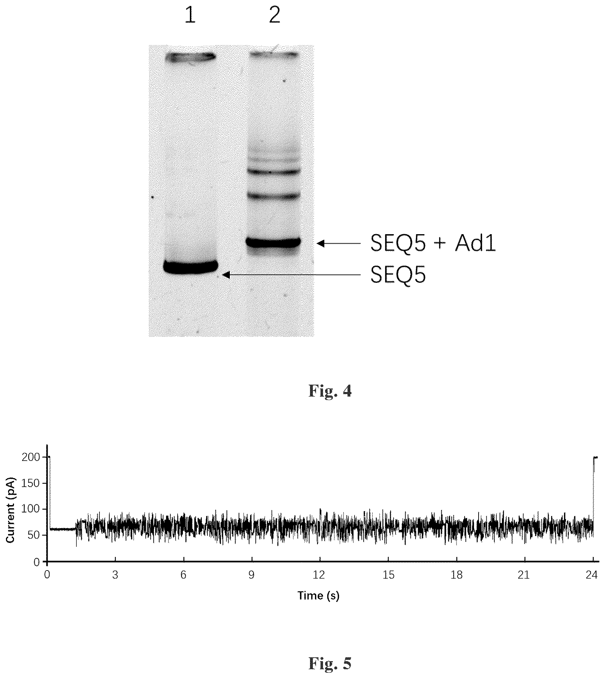

Fig. 4 shows the purification gel image after ligating the SEQ ID NO.5 that was phosphorylated with γ-methylphosphate ATP at the 5' end to Ad1, wherein Lane 1 is the template SEQ ID NO.5 sequence before ligation, Lane 2 is the product after adapter ligation and purification, and the arrow points to the product of SEQ ID NO.5 added with an Ad1 adapter.
Fig. 5 shows the typical current signal of library nanopore detection, that is, the chart of changes in the sequencing current signal after the single-channel nanopore captured the SEQ ID NO.5 sequence.

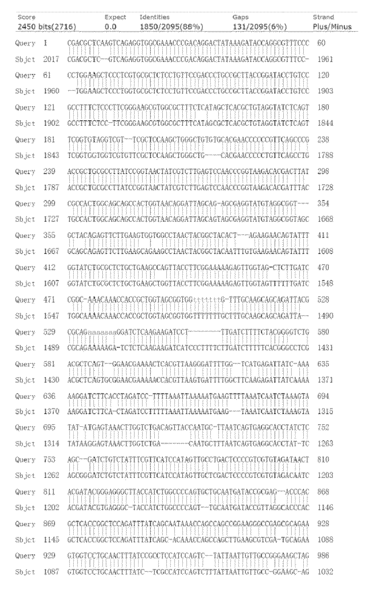

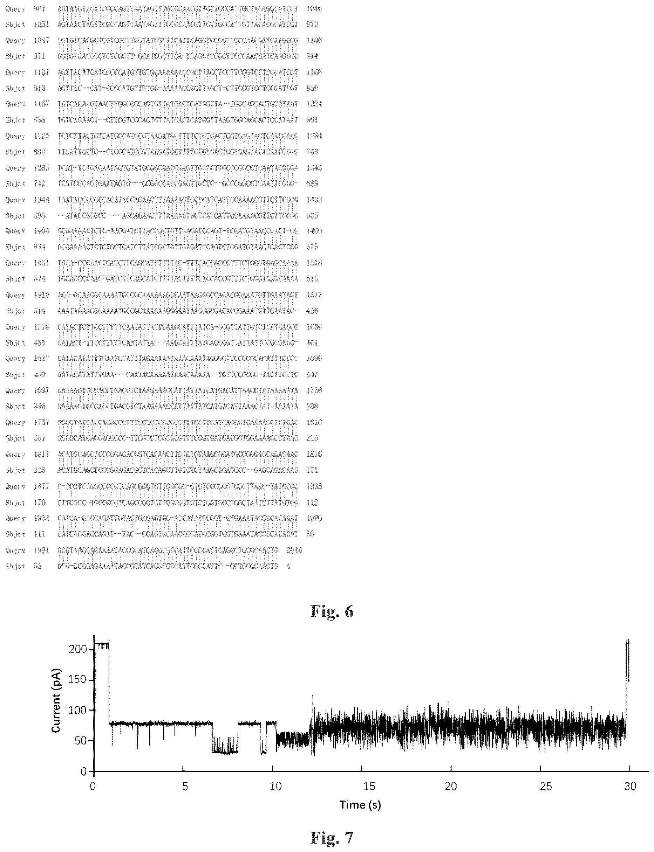

Fig. 6 shows the alignment results between the read sequence (SEQ ID NO.6, used as the Subject sequence) and the reference sequence (SEQ ID NO.5, used as the Query sequence) obtained by sequencing after the electrical signal was converted into base signal. The alignment results showed that the identity between the two sequences was 88%, and the insertion & deletion (Gap) ratio was 6%.
Fig. 7 shows the sequencing signal of the sequencing library constructed using natural ATP (negative control group). The negative control group generally only has a single-strand perforation signal, and occasionally, a nucleic acid sequence that is being unwound may be captured. Fig. 7 shows that the sequencing signal started at 12 seconds and ended at 30 seconds, that is, the duration time is shorter as compared with normal sequencing, indicating that the sequence is incomplete.

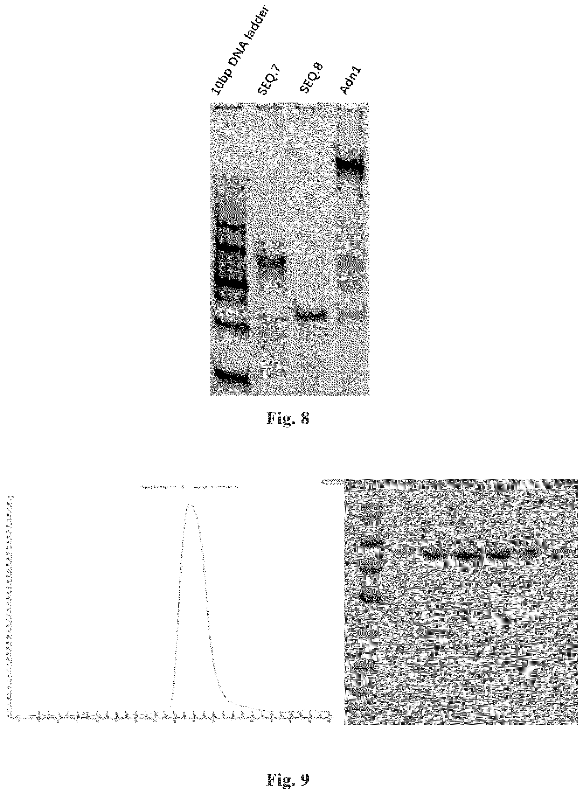

Fig. 8 shows the electropherogram of Adnl adapter annealing, wherein the lanes from left to right are: 10bp DNA Ladder (Thermo Fisher, SM1313); SEQ ID NO.7; SEQ ID NO.8; Adnl annealing product.
Fig. 9 shows the purification results of the motor protein Rep-X (SEQ ID NO. 12), wherein the left diagram shows the purification results of Rep-X using molecular sieve Superdex 200, and the right diagram shows the SDS-PAGE electrophoresis detection results.

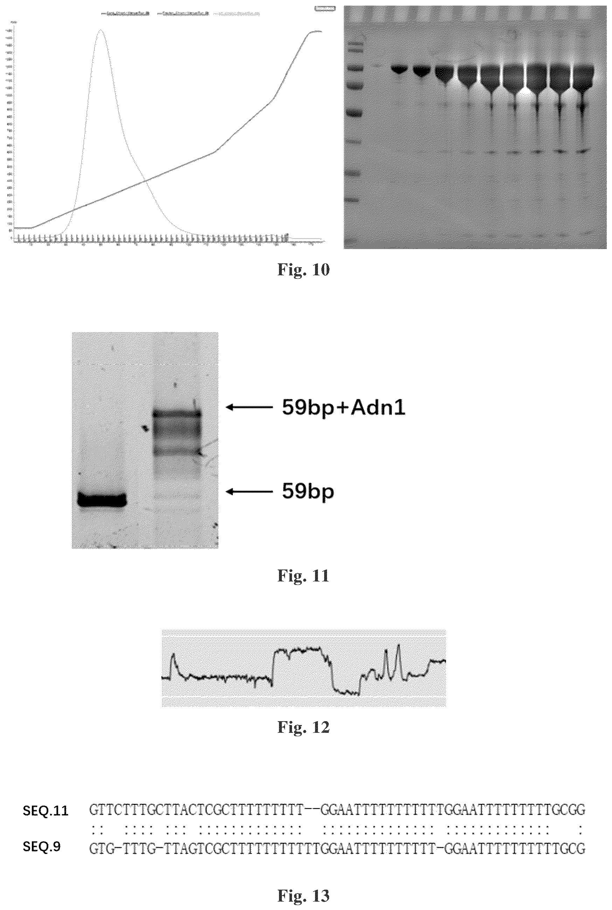

Fig. 10 shows the purification results of the polymerase mutant PGV2 (SEQ ID NO.13), wherein the left diagram shows the purification results of PGV2 protein using HiTrap Q FF anion exchange column, and the right diagram shows the SDS-PAGE electrophoresis detection results.
Fig. 11 shows the purification gel image of the sequence obtained by ligating the 59 bp sequence, into which adenosine methylphosphate was introduced at the 3' end using α-methylphosphate dATP as a reaction substrate, to the Adnl adapter, wherein the left lane is the template before ligation (lower arrow), and the right lane is the library after adapter ligation and purification (upper arrow).
Fig. 12 shows the typical current signal of library nanopore detection, in which it can be observed that the current amplitude changes have obvious sequence signal characteristics.
Fig. 13 shows the sequence alignment between the read sequence SEQ ID NO. 11 and SEQ ID NO.9, in which the SEQ ID NO.11 sequence was used as the Query sequence and the SEQ ID NO.9 sequence was used as the Subject sequence to perform a dual-sequence BLAST alignment, the sequence identity was 83.9%, and the insertion & deletion (Gap) ratio was 8.4%.

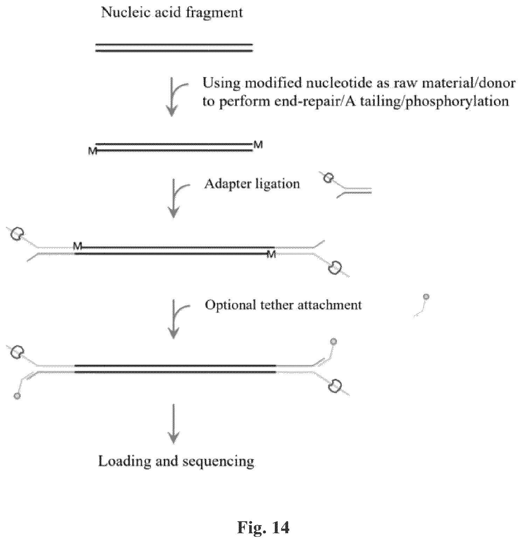

Fig. 14 shows a schematic diagram of the technical process of the present invention. The general process is as follows: using a modified nucleotide as raw material or donor to perform end-repair, addition of A or phosphorylation on a target nucleic acid fragment, then performing adapter ligation, incubating with a tether sequence (optional step), and then loading the resulting product for sequencing.

### Specific Models for Carrying Out the Invention

### Definition

The term "nucleic acid" as used herein generally refers to a molecule containing one or more nucleic acid subunits. The nucleic acid may include one or more subunits selected from adenosine (A), cytosine (C), guanine (G), thymine (T) and uracil (U), or variants thereof. Nucleotides may comprise A, C, G, T, or U, or variants thereof. Nucleotides may comprise any subunit that can be incorporated into a growing nucleic acid chain. Such subunits may be A, C, G, T, or U, or any other subunits that are specific for one or more complementary A, C, G, T, or U, or complementary to purine (i.e., A or G, or variant thereof) or pyrimidine (i.e., C, T, or U, or variant thereof). Subunits enable individual nucleic acid bases or groups of bases (e.g., AA, TA, AT, GC, CG, CT, TC, GT, TG, AC, CA, or uracil counterparts thereof) to be resolved. In some examples, the nucleic acid is deoxyribonucleic acid (DNA) or ribonucleic acid (RNA), or derivative thereof. The nucleic acid can be single-stranded or double-stranded.

The term "modified nucleotide" as used herein refers to a nucleotide bearing a modified group capable of blocking a motor protein. Preferably, such modifying group may include, but is not limited to: alkyl, fluorophore, streptavidin and/or biotin, cholesterol, methylene blue, dinitrophenol (DNP), digoxigenin ligand and/or anti-digoxigenin ligand, dibenzocyclooctynyl, etc.

The term "polymerase" as used herein generally refers to any enzyme capable of catalyzing the polymerization of bases. Examples of polymerases include, but are not limited to, nucleic acid polymerases, transcriptases, reverse transcriptases, or terminal transferases. For example, the polymerase may be *Thermococcus gorgonarius* (Tgo) polymerase or mutant thereof, *Thermus aquaticus* (Taq) polymerase I, or *Escherichia coli* polymerase I Klenow fragment, but is not limited thereto. Examples of polymerases are known in the art, and those skilled in the art can select a suitable polymerase according to actual needs.

The term "polynucleotide kinase (PNK)" as used herein generally refers to any enzyme capable of catalyzing the transfer of γ-phosphate group of a ribonucleotide to the 5'-end hydroxyl group of a target oligonucleotide. Examples of polynucleotide kinases include, but are not limited to, nucleic acid kinases, phosphotransferases, or phosphatases. For example, the polynucleotide kinase may be a T4 phage-derived polynucleotide kinase, a human-derived polynucleotide kinase 3'-phosphatase, a human-derived polynucleotide 5'-hydroxykinase NOL9, or a human-derived polynucleotide 5'-hydroxykinase Clp 1. Examples of polynucleotide kinases are also known in the art, and those skilled in the art can select appropriate polynucleotide kinases according to actual needs.

The term "motor protein" as used herein generally refers to a protein that can directly or indirectly bind to a nucleic acid and drive the nucleic acid molecule to pass through a nanopore via hydrolyzing the energy-supplying molecule. Examples of motor proteins include, but are not limited to, helicases, polymerases, ligases, or exonucleases. For example, the motor protein may be a helicase.

As used herein, the term "helicase" generally refers to an enzyme that binds to single-stranded nucleic acids and breaks the hydrogen bonds between double-stranded nucleic acid molecules by hydrolyzing the high-energy phosphate bonds of ATP molecules, thereby unwinding the double-stranded nucleic acid molecules to form single-stranded nucleic acid molecules. Examples of helicases include, but are not limited to, helicases that move in the direction from 3' to 5' of nucleic acid (e.g., helicase Rep and its mutant Rep-X), or helicases that move in the direction from 5' to 3' of nucleic acid (e.g., helicase Dda and its mutants).

In the present application, the terms "motor protein" and "helicase" are used interchangeably. Moreover, examples of motor proteins and/or helicases are known in the art, and those skilled in the art can select appropriate motor proteins and/or helicases according to actual needs.

The term "nanopore" as used herein generally refers to a pore, channel or pathway formed in a membrane or otherwise provided. The membrane may be an organic membrane, such as a lipid bilayer, or a synthetic membrane, such as a membrane formed from a polymeric material. The membrane can also be of polymeric material. Nanopores may be disposed adjacent or close to sensing circuits or electrodes coupled to sensing circuits, such as, for example, complementary metal oxide semiconductors (CMOS) or field effect transistor (FET) circuits. In some examples, nanopores have a characteristic width or diameter from about 0.1 nanometer (nm) to about 1000 nm. Some nanopores are proteins.

Taking the library construction process of Oxford Nanopore Company as an example to illustrate the existing technical solutions in this field. The process starts with genome DNA extraction, genome fragmentation is an optional operation, followed by end-repair and A-tailing at the 3' end, adapter ligation is performed, then tether sequence (tether) attachment (optional operation) is performed, and the library construction is completed (Fig. 1A). The technical process is not much different from the PCR free library construction method of conventional sequencing-by-synthesis. The main differences are: (1) the fragmentation process can be skipped to build the library directly; (2) the structure of the adapter sequence is quite different from that of sequencing-by-synthesis; and (3) the tether sequence can be picked for attachment.

In the library used for nanopore sequencing, in addition to conventional nucleic acid molecules, the adapter sequence mainly contains three special functional regions (Fig. 1B): the first functional region is a motor protein binding site, which is a segment of a single-stranded DNA sequence, usually is a polymer of thymine nucleotides with a number of 6-10; followed by the second functional region, which is a spacer sequence, in the specific embodiment of the prior art, it is 4 iSp18 polymers, of which the chemical structure is shown in Fig. 1C, and its main function is to block a motor protein and prevent it from unwinding further before sequencing; the third functional region is a binding site for a tether sequence, and its main function is to perform complementary pairing with a tether sequence, so that the tether sequence can attach to the target sequencing nucleic acid molecule in the form of non-covalent bond, thereby pulling the target sequencing nucleic acid molecule to the membrane where the sequencing pore is located, and increasing the probability of the library being sequenced.

However, in existing nanopore sequencing, the motor protein will first stay at the spacer, and then when the library moves to the nanopore, the spacer will pass through the active area of the motor protein under the action of electric field force, thereby losing the blocking effect. The motor protein then begins to unwind normally, initiating sequencing. This means that existing technical means inevitably need to detect an adapter sequence after the spacer during sequencing, and this sequence may cause crosstalk to subsequent information analysis results.

The embodiments of the present invention will be described in detail below with reference to examples, but those skilled in the art will understand that the following examples are only used to illustrate the present invention and should not be regarded as limiting the scope of the present invention. If the specific conditions are not specified in the examples, the conditions should be carried out according to the conventional conditions or the conditions recommended by the manufacturer. If the manufacturer of the reagents or instruments used is not indicated, they are all conventional products that can be purchased commercially.

### Examples

### Example 1. Preparation of Ad1 adapter sequence

In this example, the Ad1 adapter sequence was prepared by annealing the chemically synthesized SEQ ID NO.1 and SEQ ID NO.2.
1. SEQ ID NO.1 and SEQ ID NO.2 sequences (both were primers) were purchased from Sangon Biotech, and the SEQ ID NO.1 primer and SEQ ID NO.2 primer were dissolved with TE buffer (pH=8) according to the manufacturer's instructions to form stock solutions with a final concentration of 100 µM. Then, 10 µL of each of the stock solutions was taken and diluted by adding 40 µL of TE buffer (pH = 8) to form working solutions with a final concentration of 20 µM.
   SEQ ID NO.1 
5’-TTTTTTTTTTTTTTTTTTTTTTTTTTTTTTGGTTGTTTCTGTT-
GGTGCTGATATTGCT -3’
   SEQ ID NO.2
      5'pho-GCAATATCAGCACCAACAGAAACAACCTTTGAGGCGAGCGGTCAA-3'
2. 30 µL of the working solution of SEQ ID NO.1 primer and 30 µL of the working solution of SEQ ID NO.2 primer diluted in the previous step were taken respectively and mixed together, a vortex shaker was used to mix them thoroughly, and a thermal cycler was used for incubation by heating the mixture to 70°C for 10 minutes, then the temperature was lowered to 25°C at a cooling rate of 0.1°C/s and the incubation was continued for half an hour; at this point, an annealed 10 µM adapter solution was obtained, and the adapter product was named Ad1. 15% non-denaturing PAGE gel was used to detect the quality of the Ad1 product. The results were shown in Fig. 2.

### Example 2. Cloning, expression and purification of helicase Dda

In this example, helicase Dda (SEQ ID NO.4) was prepared by recombinant expression in *Escherichia coli,* and this helicase was used as a motor protein.
1. A full-length cDNA sequence of full-length Dda (SEQ ID NO.3) was purchased from Sangon Biotech, then it was ligated into a pET-28a(+) plasmid, and the double enzyme cleavage sites were Nde1 and Xho1, so that the expressed Dda protein had at its N-terminal a 6*His tag and a thrombin enzyme cleavage site.
2. The cloned pET-28a(+)-Dda plasmid was transformed into ArcticExpress(DE3) competent bacteria (Tolo Biotech., 96183-02) or derivative bacteria thereof. A single colony was picked, inoculated into 5 mL of LB medium containing kanamycin, and cultured overnight at 37°C with shaking. Then it was transferred and inoculated into 1 L of LB (containing kanamycin), cultured with shaking at 37°C until OD600=0.6-0.8, cooled to 16°C, and added with IPTG to a final concentration of 500 µM to induce Dda expression overnight.
3. Five buffer solutions were prepared according to the following formulas:
   Buffer A: 20 mM Tris-HCl pH 7.5, 250 mM NaCl, 20 mM imidazole;
   Buffer B: 20 mM Tris-HCl pH 7.5, 250 mM NaCl, 300 mM imidazole;
   Buffer C: 20 mM Tris-HCl pH 7.5, 50mM NaCl;
   Buffer D: 20 mM Tris-HCl pH 7.5, 1000 mM NaCl;
   Buffer E: 20 mM Tris-HCl pH 7.5, 100 mM NaCl;
4. The bacteria expressing Dda were collected, and the bacteria were resuspended in Buffer A, the bacteria were disrupted with a cell disruptor, and then centrifuged to take a supernatant. The supernatant was mixed with a Ni-NTA packing that had been previously equilibrated with Buffer A, and binding was performed for 1 hour. The packing was collected and washed extensively with Buffer A until no impurity proteins were washed out. Then Buffer B was added to the packing to elute Dda. The eluted Dda was passed through a desalting column that had been equilibrated with Buffer C so that the buffer was replaced. Then it was added with an appropriate amount of thrombin (Yisheng Biotechnology, 20402ES05), and loaded onto a ssDNA cellulose (Sigma, D8273-10G) packing that had been equilibrated with Buffer C, and enzymatic digestion and binding were performed overnight at 4°C. The ssDNA cellulose packing was collected, washed 3-4 times with Buffer C, and then eluted with Buffer D. The protein purified from ssDNA cellulose was concentrated and loaded onto a molecular sieve Superdex 200 (Sigma, GE28-9909-44), in which the molecular sieve buffer used was Buffer E. The target protein peak was collected, concentrated and cryopreserved. Nanodrop was used to quantify the concentration of the purified protein. HPLC and SDS-PAGE electrophoresis were used to detect the purity of the protein at the same time. The results were shown in Fig. 3.
   Nucleotide sequence of helicase Dda (SEQ ID NO.3): 
ATGACATTTGATGATTTGACCGAAGGCCAGAAAAATGCCTTTAACATT-
GTTATGAAGGC-
TATTAAAGAAAAGAAACATCATGTAACTATTAATGGACCTGCTGGTACCGGTAAG
ACTACTCTTACTAAGTTCATCATTGAAGCTTTAATATCTACGGGTGGAACTGG-
TATTATTTTAGCAGCTCCTACACATGCAGCTAAAAAGATTCTTTCAAAACTATCAG
GGAAAGAAGCGAGTACTATTCATAGTATTCTTAAAATTAACCCAGTAACAT-
ATGAA-
GAAAATGTTCTTTTTGAACAAAAAGAAGTACCTGATTTAGCCAAATGCAGAGTAT
TAATCTGCGACGAAGTGTCAATGTATGATAGAAAGC-
TATTTAAAATTCTGCTTTCAAC-
TATTCCACCTTGGTGTACTATAATTGGAATAGGGGATAATAAGCAAATCAGACCT
GTTGAACCAGGAGAAAATACTGCTTATATCAGTCCATTCTTTACACATAAAGAT-
TTTTATCAGTGTGAACTCACTGAAGTTAAACGCAGTAATGCTCCTATTATTGATGT
AGCTACTGACGTTCGCAACGGTAAGTGGAATTATGATAAAGTTGTTGACGGG-
CATGGAGTAC-
GTGGATTTACTGGTGATACCGCTTTACGCGATTTTATGGTAAATTATTTTTCAATC
GTCAAATCACTAGATGATTTGTTTGAAAATCGCGTAATGGCATTTACGAA-
TAAATCTGTT-
GACAAGTTAAATAGCATTATTCGTAAAAAGATTTTTGAAACTGATAAAGATTTTA
TTGTCGGTGAAATTATTGTAATGCAGGAACCATTATTTAAAACATATAAAATT-
GATGGAAA-
GCCTGTGTCAGAAATTATTTTTAATAACGGACAATTAGTTCGTATTATAGAAGCA
GAGTATACATCAACGTTTGTTAAAGCCCGTGGTGTTCCTGGAGAA-
TATCTAATTCGTCATT-
GGGATTTAACAGTAGAAACTTATGGCGATGATGAATATTATCGTGAAAAGATTAA
AATAATTTCATCTGACGAAGAATTGTATAAGTTTAAC-
CTATTTTTAGCTAAAACAG-
CAGAAACTTATAAAAATTGGAACAAAGGCGGAAAAGCTCCGTGGAGTGATTTTT
GGGATGCTAAATCACAGTTTAGTAAAGTGAAAGCACTTCCTG-
CATCAACATTCCATAAAGCG-
CAGGGTATGTCTGTAGACCGTGCTTTCATTTATACGCCTTGTATTCATTATGCAGA
TGTTGAATTAGCTCAACAACTTCTTTATGTT-
GGTGTCACCCGTGGTCGTTATGATGTGTTTTATGTATGA
   Amino acid sequence of helicase Dda protein (SEQ ID NO.4):

MTFDDLTEGQKNAFNIVMKAIKEKKHHVTINGPAGTGKTTLTKFIIEALISTGGT-
GIILAAPTHAAKKILSKLSGKEASTIHSILKINPVTYEENVLFEQKEVPDLAKCRVLICD
EVSMYDRKLFKILLSTIPPWCTIIGIGDNKQIRPVE-
PGENTAYISPFFTHKDFYQCELTEV-
KRSNAPIIDVATDVRNGKWNYDKVVDGHGVRGFTGDTALRDFMVNYFSIVKSLDDL
FENRVMAFTNKSVDKLNSIIRKKIFETDKDFIVGEIIVMQEPLFKTYKIDGKPVSEIIFN-
NGQLVRIIEAEYTSTFVKARGVPGEYLIRHWDLTVETYGDDEYYREKIKIISSDEELYK
FNLFLAKTAETYKNWNKGGKAPWSDFWDAKSQFSKVKAL-
PASTFHKAQGMSVDRAFIYTPCIHYADVELAQQLLYVGVTRGRYDVFYV* (* means
termination)

### Example 3. Generation of 5'-end phosphorylated sequencing library using γ-methylphosphate ATP

In this example, a fragment of pUC57 plasmid (SEQ ID NO.5) was obtained by enzymatic digestion and used as a target nucleic acid sequence for sequencing, γ-methylphosphate ATP (represented by Formula V) was used as a reaction substrate, and thus a methylphosphate group was introduced on the 5'-end nucleotide of the target nucleic acid sequence for sequencing (i.e., the 5'-end nucleoside was shown in Formula II) by a phosphorylation reaction catalyzed by a polynucleotide kinase. Then, the obtained phosphorylated end-repair product was ligated to Ad1 prepared in Example 1, and then bound to the helicase Dda prepared in Example 2.
1. The empty pUC57 plasmid was transformed into DH5alpha (Vazyme Biotech, C502-02) competent bacteria. After spreading, single clones were picked and sent for sequencing. The solution of clones that were sequenced correctly (i.e., pUC57 plasmid sequence comprised correct sequence) was supplemented with sterile glycerol to a final glycerol concentration of 50% (v/v), which was labeled and stored in a -80°C refrigerator.
2. The correctly sequenced bacterial solution was cultured in large quantities and subjected to large-scale plasmid extraction (Tiangen, DP117). Qubit dsDNA BR kit (Thermofisher, Q32853) was used to determine the concentration of the extracted plasmid, and it was labeled.
3. The double-enzyme digestion system according to Table 1 was prepared, and the system was placed on a thermal cycler, and incubated at 37°C for one hour.

**Table 1: Preparation table of double-enzyme digestion system**

| Reagent | Volume |
|---|---|
| Plasmid after large-scale extraction | 5 µg |
| 10X NE Buffer^{™} 2.1(NEB, B7202S) | 5 µL |
| Hind III (NEB, R0104L) | 1 µL |
| EcoRI (NEB, R0101L) | 1 µL |
| H₂O | Supplemented to 50µL |

4. AMPure XP magnetic beads (Beckman Coulter, A63882) was taken out from the refrigerator in advance, mixed well with shaking, and then placed at room temperature for equilibration for half an hour. 50 µL of the equilibrated magnetic beads was taken and added to the above double-enzyme digestion system, mixed well with shaking, centrifuged briefly and allowed to stand at room temperature for 10 minutes.
5. The centrifuge tube was placed on a magnetic stand for 10 minutes. When the magnetic beads were completely adsorbed to the side of the magnetic stand and the solution became completely clear, the supernatant was carefully discarded.
6. The magnetic beads were resuspended with 200 µL of 75% ethanol solution, and washed by pipetting, then the centrifuge tube was placed on the magnetic stand for 10 minutes. After the magnetic beads were completely adsorbed to the side of the magnetic stand and the solution became completely clear, the supernatant was carefully discarded.
7. The above ethanol solution washing step was repeated once, the supernatant was discarded, and the centrifuge tube was allowed to stand on a magnetic stand. After the surface of the magnetic beads became dry, the magnetic beads were resuspended by adding 22 µL of TE buffer (pH = 8), and allowed to stand at room temperature for 10 minutes.
8. The centrifuge tube was placed on a magnetic stand. After all the magnetic beads were adsorbed to the side of the magnetic stand, the supernatant was transferred to a new centrifuge tube. At this point, the purified enzyme digestion product was obtained, and its sequence was shown in SEQ ID NO.5.
pUC57 plasmid digestion product (SEQ ID NO.5): 
CGACGCTCAAGTCAGAGGTGGCGAAACCCGACAGGACTATAAAGATAC-
CAGGCGTTTCCCCCTGGAAGCTCCCTCGTGCGCTCTCCTGTTCCGACCCTGCCGCT
TACCGGATACCTGTCCGCCTTTCTCCCTTCGGGAA-
GCGTGGCGCTTTCTCATAGCTCAC-
GCTGTAGGTATCTCAGTTCGGTGTAGGTCGTTCGCTCCAAGCTGGGCTGTGTGCA
CGAACCCCCCGTTCAGCCCGACCGCTGCGCCTTATCCGGTAACTATCGTCTTGAG-
TCCAACCCGGTAAGACACGACTTATCGCCACTGGCAGCAGCCACTGGTAACAGG
ATTAGCAGAGCGAGGTATGTAGGCGGTGCTACAGAGTTCTT-
GAAGTGGTGGCCTAACTAC-
GGCTACACTAGAAGAACAGTATTTGGTATCTGCGCTCTGCTGAAGCCAGTTACCT
TCGGAAAAAGAGTTGGTAGCTCTTGATCCGGCAAACAAACCACCGCTGG-
TAGCGGTGGTTTTTTTGTTTGCAAGCAGCAGATTACGCGCAGAAAAAAAGGATCT
CAAGAAGATCCTTTGATCTTTTCTACGGGGTCTGACGCTCAGTGGAAC-
GAAAACTCAC-
GTTAAGGGATTTTGGTCATGAGATTATCAAAAAGGATCTTCACCTAGATCCTTTTA
AATTAAAAATGAAGTTTTAAATCAATCTAAAGTATATATGAGTAAACTT-
GGTCTGACAG-
TTACCAATGCTTAATCAGTGAGGCACCTATCTCAGCGATCTGTCTATTTCGTTCAT
CCATAGTTGCCTGACTCCCCGTCGTGTAGATAACTACGATACGGGAGGGCTTAC-
CATCTGGCCCCAGTGCTGCAATGATACCGCGAGACCCACGCTCACCGGCTCCAGA
TTTATCAGCAATAAACCAGCCAGCCGGAAGGGCCGAGCGCAGAAGTGGTCCTG-
CAACTTTATCCGCCTCCATCCAGTCTATTAATTGTTGCCGGGAAGCTAGAGTAAGT
AGTTCGCCAGTTAATAGTTTGCGCAACGTTGTTGCCATTGC-
TACAGGCATCGTGGTGTCAC-
GCTCGTCGTTTGGTATGGCTTCATTCAGCTCCGGTTCCCAACGATCAAGGCGAGTT
ACATGATCCCCCATGTTGTGCAAAAAA-
GCGGTTAGCTCCTTCGGTCCTCCGATCGTT-
GTCAGAAGTAAGTTGGCCGCAGTGTTATCACTCATGGTTATGGCAGCACTGCATA
ATTCTCTTACTGTCATGCCATCCGTAAGATGCTTTTCTGTGACTGGTGAG-
TACTCAAC-
CAAGTCATTCTGAGAATAGTGTATGCGGCGACCGAGTTGCTCTTGCCCGGCGTCA
 
ATACGGGATAATACCGCGCCACATAGCAGAACTTTAAAAGTGCTCATCATT-
GGAAAAC-
GTTCTTCGGGGCGAAAACTCTCAAGGATCTTACCGCTGTTGAGATCCAGTTCGAT
GTAACCCACTCGTGCACCCAACTGATCTTCAGCATCTTTTACTTTCAC-
CAGCGTTTCTGGGTGAGCAAAAACAGGAAGGCAAAATGCCGCAAAAAAGGGAAT
AAGGGCGACACGGAAATGTTGAATACTCATACTCTTCCTTTTTCAA-
TATTATTGAA-
GCATTTATCAGGGTTATTGTCTCATGAGCGGATACATATTTGAATGTATTTAGAAA
AATAAACAAATAGGGGTTCCGCGCACATTTCCCCGAAAAGTGCCACCTGAC-
GTCTAAGAAAC-
CATTATTATCATGACATTAACCTATAAAAATAGGCGTATCACGAGGCCCTTTCGT
CTCGCGCGTTTCGGTGATGACGGTGAAAACCTCTGACACATGCAGCTCCCGGA-
GAC-
GGTCACAGCTTGTCTGTAAGCGGATGCCGGGAGCAGACAAGCCCGTCAGGGCGC
GTCAGCGGGTGTTGGCGGGTGTCGGGGCTGGCTTAACTATGCGGCATCAGAG-
CAGATTGTACTGAGAGTGCACCATATGCGGTGTGAAATACCGCACAGATGCGTAA
GGAGAAAATACCGCATCAGGCGCCATTCGCCATTCAGGCTGCGCAACTG
9. γ-Methylphosphate ATP was synthesized, its structural formula was shown in Formula V, and it was dissolved in ultrapure water to prepare a stock solution with a concentration of 100 mM. 10 µL of the stock solution was taken, and added with 90 µL of ultrapure water for dilution to obtain a working solution with a concentration of 10 mM.
10. A solution for 5'-end phosphorylation and end-adding A reaction was prepared on ice according to Table 2 below. After preparation, it was placed on a thermal cycler and incubated at 37°C for 30 minutes and 72°C for 30 minutes.

**Table 2: Formulation of a solution for phosphorylation and end-adding A reaction**

| Reagent | Volume |
|---|---|
| 10X ThermoPol Reaction Buffer (NEB B9004S) | 5 µL |
| Purified enzyme digestion product (SEQ ID NO.5) | 2 µg |
| Klenow Fragment (3'→5' exo-) (NEB, M0212L) | 2 µL |
| 100mM dATP (Enzymatics, N2010-A-L) | 0.5 µL |
| 10mM γ-methylphosphate ATP (shown in Formula V) | 5 µL |
| Polynucleotide kinase (NEB, M0201L) | 2 µL |
| 10X polynucleotide kinase reaction buffer (NEB, B0201S) | 5 µL |
| H₂O | Supplemented to 50µL |

11. AMPure XP (Beckman Coulter, A63882) magnetic beads were taken from the refrigerator in advance, mixed well with shaking, and then placed at room temperature for equilibration for half an hour. 50 µL of the equilibrated magnetic beads were taken and added to the above phosphorylation and end-adding A reaction system, mixed well with shaking, centrifuged briefly and allowed to stand at room temperature for 10 minutes.
12. The centrifuge tube was placed on a magnetic stand for 10 minutes. When the magnetic beads were completely adsorbed to the side of the magnetic stand and the solution became completely clear, the supernatant was carefully discarded.
13. The magnetic beads were resuspended using 200 µL of 75% ethanol solution and washed by pipetting. The centrifuge tube was placed on a magnetic stand for 10 minutes. When the magnetic beads were completely adsorbed to the side of the magnetic stand and the solution became completely clear, the supernatant was carefully discarded.
14. The above ethanol solution washing step was repeated once, the supernatant was discarded, and the centrifuge tube was allowed to stand on a magnetic stand. After the surface of the magnetic beads became dry, the magnetic beads were resuspended by adding 22 µL of TE buffer (pH = 8), and allowed to stand at room temperature for 10 minutes.
15. The centrifuge tube was placed on a magnetic stand. After all the magnetic beads were adsorbed to the side of the magnetic stand, 21 µL of the supernatant was transferred to a new centrifuge tube. At this point, the purified end-repair product of SEQ ID NO.5, of which 5'-end nucleotide was phosphorylated by methylphosphate group, was obtained, and 1 µL of the purified product was taken and quantified using Qubit dsDNA HS kit (Thermo Fisher, Q32854).
16. 5X ligation buffer of 330 mM Tris, 50 mM MgCl₂, 5 mM DTT, 30% PEG6000, pH 7.6 was prepared.
17. The ligation system was prepared on ice according to Table 3 below, in which Gamma-S ATP only supplied energy to the ligase and itself would not be consumed by motor protein. The system was placed on a thermal cycler and incubated at 25°C for 30 minutes.

**Table 3: Ligation system formula**

| Reagent | Volume |
|---|---|
| 5X ligation buffer | 10 µL |
| 10mM Gamma-S ATP (Sigma-Aldrich, A1388-5mG) | 5 µL |
| Phosphorylated end-repair product of Example 3 | 20 µL |
| Ad1 adapter of Example 1 | 5 µL |
| T4 DNA ligase (NEB M0202T) | 5 µL |
| H₂O | 5 µL |

18. AMPure XP magnetic beads (Beckman Coulter, A63882) were taken from the refrigerator in advance, mixed well with shaking, and placed at room temperature for at least half an hour for equilibration. After equilibration, 20 µL of the magnetic beads were taken and added to a new centrifuge tube. The centrifuge tube was placed on a magnetic stand for 10 minutes. When the magnetic beads were completely adsorbed to the side of the magnetic stand and the solution became completely clear, the supernatant was carefully discarded.
19. AMPure XP (Beckman Coulter, A63882) magnetic beads were taken from the refrigerator in advance, mixed well with shaking, and then placed at room temperature for equilibration for half an hour. After equilibration, 20 µL of the magnetic beads were taken and added to the above phosphorylation and end-adding A reaction system, mixed well with shaking, centrifuged briefly and allowed to stand at room temperature for 10 minutes.
20. The centrifuge tube was placed on a magnetic stand for 10 minutes. When the magnetic beads were completely adsorbed to the side of the magnetic stand and the solution became completely clear, the supernatant was carefully discarded.
21. The magnetic beads were resuspended using 200 µL of 75% ethanol solution and washed by pipetting. The centrifuge tube was placed on a magnetic stand for 10 minutes. When the magnetic beads were completely adsorbed to the side of the magnetic stand and the solution became completely clear, the supernatant was carefully discarded.
22. The above ethanol solution washing step was repeated once, the supernatant was discarded, and the centrifuge tube was allowed to stand on a magnetic stand. After the surface of the magnetic beads became dry, the magnetic beads were resuspended by adding 22 µL of TE buffer (pH = 8), and allowed to stand at room temperature for 10 minutes.
23. The centrifuge tube was placed on a magnetic stand. After all the magnetic beads were adsorbed to the side of the magnetic stand, 21 µL of the supernatant was transferred to a new centrifuge tube. At this point, the purified ligation product was obtained. 1 µL of the purified product was taken and quantified using Qubit dsDNA HS kit (Thermo Fisher, Q32854).
24. 3 µL of the elution product was taken for non-denaturing polyacrylamide gel electrophoresis detection, as shown in the lane 2 of Fig. 4 (wherein, SEQ5+Ad1 represents the product of ligating SEQ ID NO.5 that was end-repaired by phosphorylation with methylphosphate group to Ad1).
25. 100 mL of 1M Tris-HCL pH 7.5 buffer and 100 mL of 1M KCl solution in a volumetric flask was added with ultrapure water to adjust the volume to 1L to obtain a 2X binding buffer.
26. A mixed solution of helicase Dda (prepared in Example 2) and a library was prepared on ice according to Table 4 below, and then incubated at 30°C for one hour.

**Table 4: Binding system of motor protein and library**

| Reagent | Volume |
|---|---|
| 2 X binding buffer | 50 µL |
| Helicase Dda | 20 µL |
| Purified ligation product | 20 µL |
| H₂O | 10 µL |

27. The library was quantified using Qubit DNA HS kit. After clearly labeling the concentration, the product was stored in a 4°C refrigerator for later use.

### Example 4. Nanopore sequencing

In this example, a nanopore detection platform was constructed based on the patch clamp platform, and used to perform nanopore sequencing on the target sequencing library prepared in Example 3 (the 5' end was phosphorylated by methylphosphate group) to verify the advantages of the sequencing library that was constructed by the present application and capable of blocking a motor protein in nanopore sequencing.
1. By referring to the single-channel electrophysiological detection system as described by Geng Jia and Guo Peixuan ("Membrane-embedded channel of bacteriophage phi29 DNA-packaging motor for single molecule sensing and nanomedicin". Chinese Bulletin of Life Sciences, 2011, 23(11): 1114-1129), a nanopore detection platform based on the patch clamp platform was constructed, and porin (Sigma-Aldrich, H9395-5mg) was inserted into the phospholipid bilayer membrane to form a single-channel nanopore.
2. The sequencing library obtained in Example 3 was added to the single-channel system, and the changes in current amplitude were detected and recorded by the patch clamp system.
3. The typical SEQ ID NO.5 sequencing current diagram was shown in Fig. 5.
4. The base identification software was used to convert the changes in sequencing current amplitude into the read sequence SEQ ID NO.6, the SEQ ID NO.5 sequence was used as the Query sequence, the SEQ ID NO.6 sequence was used as the Subject sequence, the BlastN algorithm was used to perform pairwise sequence alignment, and the alignment results were shown in Fig. 6.
   The alignment results in Fig. 6 showed that the identity of the two sequences was 88%, and the insertion & deletion (Gap) ratio was 6%. The identity and the insertion & deletion ratio were comparable to those of the commercial nanopore sequencing platform (Oxford Nanopore). However, the sequencing of this protocol would not detect the fixed sequence carried by the adapter, thereby reducing crosstalk, and showing an advantage over the libraries that did not contain the modified nucleotide.
   Read sequence (SEQ ID NO.6): 
ACGCAGTTGCGCAGCGAATGGCGAATGGCGCCTGATGCGG-
TATTTTCTCCGCCG-
CATCTGTGCGGTATTTCACCACCGCATGCCGTTGCACTCGGTAATCTGCTCCTGAT
GCCACATAAGATTAGCCAGCCACCAGACACCGCCAACACCCGCTGAC-
GCGCCAGCCGAA-
GCTTGTCTGCTCGGCATCCGCTTACAGACAAGCTGTGACCGTCTCCGGGAGCTGC
ATGTGTCAGGGTTTTCCACCGTCATCACCGAAACGCGCGAGAC-
GAAGGGCCTCGTGATGCGCCTATTTTATAGTTTAATGTCATGATAATAATGGTTTC
TTAGACGTCAGGTGGCACTTTTCCAGGAAGTAGCGCGGAACATATTT-
GTTTATTTTTC-
TATTGTTCAAATATGTATCGCTCGCGGAATAATAACCCCTGATAAATGCTTTAATA
 
TTGAAAAAGGAAAGTATGGTATTCAACATTTCCGTGTCGCCCTTATTCCCTTTTTT-
GCGG-
CATTTTGCCTTCTATTTTTTTGCTCACCCAGAAACGCTGGTGAAAAGTAAAAGATG
CTGAAGATCAGTTGGGGTGCACGGAGTGAGTTACATCCAGACTG-
GATCTCAACAGCGATAA-
GATCAGCAGAGAGTTTTCGCCCCGAAGAACGTTTTCCAATGATGAGCACTTTTAA
AGTTCTGCTGGCGCGGTATCCCGTATTGACGCCGGGCGAG-
CAACTCGGTCGCCGCCAC-
TATTCACTGGGACGACCCGGTTGAGTACTCACCAGTCACAGAAAAGCATCTTACG
GATGGCAGCAGCAATGAAATTATGCAGTGCTGCCACTTAACCATGAGTGA-
TAACACTGCGAC-
CAACACTTCTGACAACGATCGGAGGACCGAAGAGCTAACCGCTTTTTGCACAACA
TGGGGATCGTAACTCGCCTTGATCGTTGGGAACCGGAGCTGATGAA-
GCCATGCAA-
GCGACAGGCGTGACACCACGATGCCTGTAACAATGGCAACAACGTTGCGCAAAC
TATTAACTGGCGAACTACTTACTCTGCTTCCGGCAACAATTAATAAAGACTG-
GATGGCGAGA-
TAAAGTTGCAGGACCACTTCTGCATCGACGCTTCAAGCTGGCTGGTTTGTGCTGA
TAAATCTGGAGCCGGTGAGCGTGGGTGCCTAACGGTATCATT-
GCAACTGGGGCCAGATGG-
TAGCCCTCACGTATCGTGATTGTCTACACGACGGGGAGTCGAGCAACTATGGATG
AACGAAATAGACAGATCCCGCTGAATAGGTGCCTCACTGATTAAAGCATT-
GTCAGAC-
CAAGTTTACTCCTTATATACTTTAGATTGATTTACTTCATTTTTAATTTAAAAAGG
ATCTAGTGAAGATCCTTTTTTGATAATCTCTTGAAGCCAAAATCACTTAAC-
GTGGTTTTTCGTTCCGCACTGAGCGTCGAGGCCCGTGAAAAAGATCAAGAAAAGG
GATGATCTTCTTGAGAGATCTTTTTCTGCGTAATCTGCTGCTTGCAAA-
GCAAAAAAACCAC-
CGCTACCAGCGGTGGTTTGTTTTGCCAGATCAAAAAACTACCAACTCTTTTTCCGA
AGGTAACCAGCTTCAGCAGAGCGCAGATACCAAATACTGTTCTTCACAAATT-
GTAGCCGTAG-
TTAGGCTTCTGCTTCAAGAACTCTGCTGCGCTACCGCCTACATACCTCGCTACTGC
 
TAATCCTGTTACCAGTGGCTGCTGCCAGTGGCAGTAAATCGTGTCTTACCGGGTT-
GGACTCAAGACGATAGTTACCGGATAAGGCGCAGCGGTCAGGCTGAACGGGGG
TTCGTGCAGCCCAGCTTGGAGCGAACACGACCACCACCGAACTGAGATAC-
CTACAGCGTGAGCGCTATGAAAGCGCCACGCTTCCCGAAGGAGAAAGGCGGACA
GGTATCCGGTAAGCGGCAGGGTCGGAACAGGAGAGCGCAC-
CAGGGAGCTTCCAGGAAAC-
GCCTGGTATCTTTATAGTCCTGTCGGGTTTCGCCACCTCTGACGAGCGTCG
5. A sequencing nucleic acid sequence, which was constructed using the same method as described above using ATP with natural structure, was used as a negative control group for sequencing. A large number of signals of single-stranded DNA passing through pores were observed, and sequencing signals were basically not be observed. Occasionally, there were signals of sequences that were being unwound and captured by nanopores, and only the current oscillation signals of a partial sequence of SEQ ID NO.5 were recorded, as shown in Fig. 7. Fig. 7 showed that the sequencing signal started at 12 seconds and ended at 30 seconds. Compared with Fig. 5, the sequencing duration was shorter and the sequence was incomplete.

Comparing the results of Fig. 7 with the results of Fig. 5, it was fully proved that because a methylphosphate group was introduced through phosphorylation at the 5' end of the target nucleic acid sequence (i.e., the 5'-end nucleotide became a modified nucleotide capable of blocking a motor protein), the nucleic acid sequencing library constructed using the method of the present invention could block the perforation speed of motor protein, avoid the detection of an adapter sequence after the spacer during sequencing, reduce the crosstalk of the adapter sequence to subsequent analysis, and at the same time enhance the signal intensity of special sequences, thereby providing help for base identification tools to better determine the starting point of a sequencing sequence.

### Example 5. Preparation of Adnl adapter

In Examples 5-9, polymerases were used to introduce adenosine methylphosphate at the 3' end of the target sequencing nucleic acid molecule to construct a sequencing library, and nanopore sequencing was performed to verify the sequencing effect, which involved the preparation of compatible adapters, motor proteins and polymerases.
1. SEQ ID NO.7 primer and SEQ ID NO.8 primer were purchased from Sangon Biotech, and the SEQ ID NO.7 primer and SEQ ID NO.8 primer were dissolved in TE buffer (pH = 8) according to the manufacturer's instructions to form stock solutions with a final concentration of 100 µM. Then 10 µL of each of the stock solutions was taken and added to 40 µL of TE buffer (pH = 8) for dilution to form working solutions with a final concentration of 20 µM.
   SEQ ID NO.7: 
5’pho-CGTTATAGTCGTGGTTGTCTTTGTT-
GGTTTTTTTTTTTTTTTTTTTTTTTTTTTTTT -3’
   SEQ ID NO.8:
      5'-AACTGGCGAGCGGAGTTTCCAACAAAGACAACCACGACTATAACGT -3'
2. 30 µL of the working solution of SEQ ID NO.7 primers and 30 µL of the working solution of SEQ ID NO.8 primers obtained by dilution in the previous step were taken respectively, mixed together, mixed thoroughly using a vortex shaker, and heated to 70°C and incubated for 10 minutes using a thermal cycler, then the temperature was lowered to 25°C at a cooling rate of 0.1°C/s and the incubation was continued for half an hour. At this point, a 10 µM adapter solution was obtained, and the adapter was named Adnl, as shown in Fig. 8.

### Example 6. Cloning, expression and purification of helicase Rep-X

In this example, helicase Rep-X was prepared by recombinant expression in *Escherichia coli* and used as a motor protein.

### 1. Cloning and expression of Rep-X

A full-length Rep-X cDNA sequence was purchased from Sangon Biotech and ligated into a pET-28a(+) plasmid, and the used double enzyme cleavage sites were Nde1 and Xho1, so that the N-terminal of the expressed Rep-X protein had 6 *His tag and thrombin restriction site.

The cloned pET-28a(+)-Rep-X plasmid was transformed into *Escherichia coli* expression strain BL21(DE3). A single colony was picked, inoculated into 5 mL of LB medium containing kanamycin, and cultured overnight at 37°C with shaking. Then it was transferred and inoculated into 1 L of LB, cultured with shaking at 37°C until OD600=0.6-0.8, cooled to 16°C, and added with IPTG to a final concentration of 500 µM to induce expression overnight.

### 2. Rep-X purification

Five buffer solutions were prepared according to the following formulas:
Buffer A': 20 mM Tris-HCl pH 8.0, 250 mM NaCl, 20 mM imidazole
Buffer B': 20mM Tris-HCl pH 8.0, 250mM NaCl, 300mM imidazole
Buffer C': 20 mM Tris-HCl pH 8.0, 50 mM NaCl
Buffer D': 20 mM Tris-HCl pH 8.0, 1000 mM NaCl
Buffer E': 20 mM Tris-HCl pH 7.0, 100 mM NaCl

The expressed Rep-X bacterial cells were collected, the bacterial cells were resuspended in Buffer A', the bacterial cells were disrupted with a cell disruptor, and then centrifuged to take the supernatant. The supernatant was mixed with a Ni-NTA packing that had been previously equilibrated with Buffer A', and binding was performed for 1 hour. The packing was collected and washed extensively with Buffer A' until no impurity protein was washed out. Then Buffer B' was added to the packing to elute Rep-X. The eluted Rep-X protein was passed through a HiTrap Desalting column (Cytiva, 29048684) that had been previously equilibrated with Buffer C', so that the buffer was replaced. Then an appropriate amount of thrombin (Yisheng Biotech, 20402ES05) was added, and enzymatic digestion was carried out overnight at 4°C. The digested Rep-X was purified using a HiTrap Q FF anion exchange column (Cytiva, 17505301). The Q column was equilibrated with Buffer C', then the sample was loaded. After sufficiently washing with Buffer C', Rep-X was separated and purified by running in a continuous gradient from Buffer C' to Buffer D'. The protein purified by the Q column was concentrated and loaded onto a molecular sieve Superdex 200 (Sigma, GE28-9909-44), in which the molecular sieve buffer used was Buffer E'. The molecular sieve peak diagram and SDS-PAGE results of the finally purified protein were shown in Fig. 9. The target protein peak was collected, concentrated and cryopreserved.

Amino acid sequence of helicase Rep-X (SEQ ID NO. 12)

mrlnpgqqqavefvtgpllvlagagsgktrvitnkiahlirgsgyqarhiaavtft-
nkaaremkervgqtlgrkeargImistfhtlgldiikreyaalgmkanfslfddtdqlallkelteglieddkvllqqlistisnwkndl
ktpsqaaasaigerdrifahvyglydahlkacnvldfddlillptlllqrneevrkrwqn-
kiryllvdeyqdtntsqyelvkllvgsrarftvvgdddqsiyswrgarpqnlvllsqdfpalkvikleqnyrssgrilkaanilianmph

vfekrlfselgygaelkvlsanneeheaervtgeliahhfvnktqykdyailyr-
gnhqsrvfekflmqnripykisggtsffsrpeikdllaylrvltnpdddcaflrivntpkreigpatlkklgewamtrnksmftasfd
mglsqtlsgrgyealtrfthwlaeiqrlaerepi-
aavrdlihgmdyeswlyetspspkaaemrmknvnqlfswmtemlegseldepmtltqvvtrftlrdmmergeseeeldqvql
mtlhaskglefpyvymvgmeegflphqssidednideerrlayvgitraqkeltftlak-
errqygelvrpepsrfllelpqddliweqerkvvsaeermqkgqshlanlkammaakrgk

### Example 7. Cloning, expression and purification of polymerase mutant PGV2

In this example, polymerase mutant PGV2 (SEQ ID NO. 13) was prepared by recombinant expression in *Escherichia coli.*

### 1. Cloning and expression of PGV2

PGV2 was a reported *Thermococcus gorgonarius* polymerase mutant with the ability to polymerize methylphosphate dNTP (Sebastian Arangundy-Franklin et al, Nature Chemistry, vol. 11, pages533-542 (2019), see https://doi.org/ 10.1038/s41557-019-0255-4). The full-length cDNA sequence of polymerase mutant PGV2 was purchased from Sangon Biotech, and ligated into pET-28a(+) plasmid. The used double enzyme cleavage sites were Ncol and Xho1, so that the C-terminal of the expressed protein had a 6*His tag.

The cloned pET-28a(+)-PGV2 plasmid was transformed into *Escherichia coli* expression strain BL21(DE3) or derivative bacteria thereof. A single colony was picked, inoculated into 5 mL of LB medium containing kanamycin, and cultured overnight at 37°C with shaking. Then it was transferred and inoculated into 1 L of LB, cultured with shaking at 37°C until OD600=0.6-0.8, cooled to 16°C, and added with IPTG to a final concentration of 500 µM to induce expression overnight.

### 2. Purification of PGV2

Five buffer solutions were prepared according to the following formulas:
Buffer F: 20 mM Tris-HCl pH 7.5, 250 mM NaCl, 20 mM imidazole;
Buffer G: 20 mM Tris-HCl pH 7.5, 250 mM NaCl, 300 mM imidazole;
Buffer H: 20 mM Tris-HCl pH 8, 50 mM NaCl;
Buffer I: 20 mM Tris-HCl pH 8, 1000 mM NaCl;
Buffer J: 20 mM Tris-HCl pH 7.5, 100 mM NaCl;

The expressed PGV2 bacterial cells were collected, the bacterial cells were resuspended in Buffer F, the bacterial cells were disrupted with a cell disruptor, and then centrifuged to take the supernatant. The supernatant was mixed with a Ni-NTA packing that had been previously equilibrated with Buffer F, and binding was performed for 1 hour. The packing was collected and washed extensively with Buffer F until no impurity protein was washed out. Buffer G was then added to the packing to elute PGV2. The eluted PGV2 protein was passed through a HiTrap Desalting column that had been previously equilibrated with Buffer H, so that the buffer was replaced. Then it was loaded onto a HiTrap Q FF anion exchange column that had been equilibrated with Buffer H. After being fully washed with Buffer H, elution was carried out by running in a continuous gradient from Buffer H to Buffer I. The purified protein was concentrated and loaded onto a desalting column, in which the buffer used was Buffer J. The anion column elution and SDS-PAGE results of the finally purified protein were shown in Fig. 10. The target protein peak was collected, concentrated and cryopreserved.

Amino acid sequence of polymerase mutant PGV2 (SEQ ID NO.13)

mildtdyitedgkpvirifkkengefkidydrnfepyiyallkddsaiedvkki-
taerhgttvrvvraekvkkkflgrpievwklyfthpqdqpairdkikehpavvdiyeydipfakrylidkglipmegdeelkmlaf
aiatlyhegeefaegpilmisyadeegarvit-
wknidlpyvdvvstekemikrflkvvkekdpdvlityngdnfdfaylkkrseklgvkfilgregsepkiqrmgdrfavevkgrih
fdlypvirrtinlptytleavyeaifgqpkekvyaeeiaqawetgeg-
lervarysmedakvtyelgkeffpmeaqlsrlvgqslwdvsrsstgnlvewfllrkayernelapnkpderelarrresyaggyvk
epergiwenivyldfrslypsiiithnvspdtlnregcgeydvapqvghkfckdfpgfip-
sllgplleerqkvkkkmkatidpiekklldyrqrligilansfygyygyakarwyckecaesvtawgrqylettireieekfgfkvly
adtdgffatipgadaetvkkkakefldyinaklpglleleyegfykrgffvtkkkya-
videedkittvgleivrvdwseiaketqarvleailkhgdveeavrivkevteklskyevppeklviyeqitrdlkdykatgphvava
krlaargikirpgtvisyivlkgsgrigdraipfdefdpakhrydaeyyienqvlpaver-
ilrafgyrkedlryqktrqvglgawlkpkt

### Example 8. Construction of sequencing library of target sequencing nucleic acid molecule introduced at its 3' end one adenosine methylphosphate

In this example, SEQ ID NO.9 and SEQ ID NO.10 were annealed and ligated to obtain a 59 bp sequence, which was used as a target sequencing nucleic acid molecule to construct a target sequencing library for nanopore sequencing. The polymerase mutant PGV2 obtained in Example 7 was used to introduce one adenosine methylphosphate (shown in Formula III) at the 3' end of the target sequencing nucleic acid sequence. Then, the obtained phosphorylated end-repair product was ligated to the Adnl adapter prepared in Example 5, and then bound to the helicase Rep-X prepared in Example 6.
1. SEQ ID NO.9 primer and SEQ ID NO.10 primer were purchased from Sangon Biotech (the two were completely complementary), and the SEQ ID NO.9 primer and SEQ ID NO.10 primer were dissolved in TE buffer (pH = 8) according to the manufacturer's instructions to form stock solutions with a final concentration of 100 µM. Then 10 µL of each of the stock solutions were taken respectively, and added with 40 µL of TE buffer (pH = 8) for dilution to obtain working solutions with a final concentration of 20 µM.
SEQ ID NO.9: 
5’-GTGTTTGTTAGTCGCTTTTTTTTTTTGGAATTTTTTTTTT-
GGAATTTTTTTTTTGCGCT -3’
SEQ ID NO.10:

5'-AGGCAAAAAAAAAATTCCAAAAAAAAAAATTCCAAAAAAAAAAA-
GCGACTAACAAACAC -3'

2. 30 µL of the working solution of SEQ ID NO.9 primers and 30 µL of the working solution of SEQ ID NO.10 primers obtained by dilution in the previous step were taken respectively, mixed together, mixed thoroughly with shaking using a vortex shaker, and heated to 70°C and incubated for 10 minutes using a thermal cycler, then the temperature was lowered to 25°C at a cooling rate of 0.1°C/s and the incubation was continued for half an hour. At this point, a 10 µM 59 bp library template was obtained.
3. Design and synthesis of α-methylphosphate dATP, as shown in Formula VII.
4. A mixed solution for the end-repair was prepared according to Table 5, placed on a thermal cycler, and incubated at 37°C for 30 minutes and 72°C for 30 minutes to perform end-repair and phosphorylation, so that one adenosine methylphosphate (shown in Formula III) was added to the 3' end of the target sequencing nucleic acid sequence.

**Table 5: Formula of end-repair system**

| Reagent | Volume |
|---|---|
| 100 µM library template (prepared in Example 8) | 20 µL |
| 10 mM α-methylphosphate dATP (as shown in Formula VII) | 5 µg |
| 10X ThermoPol reaction buffer (NEB) | 5 µL |
| 0.05 µM polymerase mutant PGV2 (prepared in Example 7) | 5 µL |
| 10 mM ATP (Enzymatics, N2070-A-L) | 5 µL |
| Polynucleotide kinase (NEB, M0201L) | 2 µL |
| 10X Polynucleotide kinase buffer (NEB, B0201S) | 5 µL |
| H₂O | 3 µL |

5. AMPure XP magnetic beads (Beckman Coulter, A63882) were taken from the refrigerator in advance, mixed well with shaking, and placed at room temperature for at least half an hour for equilibration. 50 µL of the equilibrated magnetic beads were added to the above-mentioned end-repair system, mixed well with shaking, centrifuged briefly and allowed to stand at room temperature for 10 minutes.
6. The centrifuge tube was placed on a magnetic stand for 10 minutes. When the magnetic beads were completely adsorbed to the side of the magnetic stand and the solution became completely clear, the supernatant was carefully discarded.
7. The magnetic beads were resuspended using 200 µL of 75% ethanol solution and washed by pipetting. The centrifuge tube was placed on a magnetic stand for 10 minutes. After the magnetic beads were completely adsorbed to the side of the magnetic stand and the solution became completely clear, the supernatant was carefully discarded.
8. The above ethanol solution washing step was repeated once, the supernatant was discarded, and the centrifuge tube was allowed to stand on a magnetic stand. After the surface of the magnetic beads became dry, the magnetic beads were resuspended by adding 21 µL of molecular grade water, and allowed to stand at room temperature for 10 minutes.
9. The centrifuge tube was placed on a magnetic stand. After all the magnetic beads were adsorbed to the side of the magnetic stand, the supernatant was transferred to a new centrifuge tube. At this point, the purified end-repair product was obtained.
10. 1 µL of the purified product was taken and quantified using Qubit dsDNA HS kit (Thermo Fisher, Q32854), and the remaining product was used for the next reaction.
11. 5X ligation buffer of 330 mM Tris, 50 mM MgCl₂, 5 mM DTT, 30% PEG6000, pH 7.6 was prepared.
12. The ligation system was prepared on ice according to Table 6. The system was placed on a thermal cycler and incubated at 25°C for 30 minutes.

**Table 6: Formula of ligation system**

| Reagent | Volume |
|---|---|
| 5X ligation buffer | 10 µL |
| 10mM Gamma-S ATP (Sigma-Aldrich, A1388-5mG) | 5 µL |
| End-repair product | 20 µL |
| Adnl adapter (prepared in Example 5) | 5 µL |
| T4 DNA ligase (NEB M0202T) | 5 µL |
| H₂O | 5 µL |

13. AMPure XP magnetic beads (Beckman Coulter, A63882) were taken from the refrigerator in advance, mixed with shaking, and placed at room temperature for at least half an hour for equilibration. After equilibration, 20 µL of the magnetic beads were taken and added into a new centrifuge tube. The centrifuge tube was placed on a magnetic stand for 10 minutes. When the magnetic beads were completely adsorbed to the side of the magnetic stand and the solution became completely clear, the supernatant was carefully discarded.
14. The magnetic beads were resuspended using 20 µL of the magnetic bead-binding buffer 1 and washed by pipetting. The centrifuge tube was placed on a magnetic stand for 10 minutes, when the magnetic beads were completely adsorbed to the side of the magnetic stand and the solution became completely clear, the supernatant was carefully discarded.
15. The above washing step with magnetic bead-binding buffer 1 was repeated once. After the supernatant was discarded, 20 µL of magnetic bead-binding buffer 1 was added to the magnetic beads and kept at room temperature for later use.
16. The above magnetic beads were added to the ligation system and mixed evenly using a pipette to avoid the generation of air bubbles.
17. The magnetic bead purification system in the previous step was placed on a magnetic stand for 10 minutes. After the magnetic beads were completely adsorbed to the side of the magnetic stand and the solution became completely clear, the supernatant was carefully discarded.
18. The magnetic beads were resuspended using an equal volume of magnetic bead-binding buffer 2 and washed by pipetting. The centrifuge tube was placed on a magnetic stand for 10 minutes. When the magnetic beads were completely adsorbed to the side of the magnetic stand and the solution became completely clear, the supernatant was carefully discarded.
19. The above washing step with the magnetic bead-binding buffer 2 was repeated once, the supernatant was discarded, and the magnetic beads were resuspended by adding elution buffer. The resuspended magnetic beads were allowed to stand at room temperature for 10 minutes, then the centrifuge tube was placed on a magnetic stand. When all the magnetic beads were adsorbed to the side of the magnetic stand, the supernatant was transferred to a new centrifuge tube. At this point, the purified ligation product was obtained. The ligation product was a sequence formed by ligating the 59 bp sequence, in which adenosine methylphosphate was introduced at the 3' end, to the Adnl adapter, and its purification gel image was shown in Fig. 11.
20. 100 mL of 1M Tris-HCL pH 7.5 buffer and 100 mL of 1M KCl solution were added to a volumetric flask, and ultrapure water was added to adjust the volume to 1L, thereby obtaining a 2X binding buffer.
21. The mixed solution of the helicase Rep-X obtained in Example 6 and the ligation product were prepared on ice according to Table 7 below, and then incubated at 30°C for 1 hour.

**Table 7: System for binding motor protein to library**

| Reagent | Volume |
|---|---|
| 2X binding buffer | 50 µL |
| Helicase Rep-X | 20 µL |
| Purified ligation product | 20 µL |
| H₂O | 10 µL |

22. Qubit DNA HS kit was used to quantify the obtained sequencing library. After the concentration was labeled clearly, the product was stored in a 4°C refrigerator for later use.

### Example 9. Nanopore sequencing

In this example, a nanopore detection platform was constructed based on a patch clamp platform, and nanopore sequencing was performed on the target sequencing sequence prepared in Example 8 (one adenosine methylphosphate was introduced at the 3' end) to verify the advantages of the sequencing library, which was constructed in the present application and capable of blocking a motor protein, in nanopore sequencing.
1. By referring to the single-channel electrophysiology as described by Geng Jia and Guo Peixuan ("Membrane-embedded channel of bacteriophage phi29 DNA-packaging motor for single molecule sensing and nanomedicin". Chinese Bulletin of Life Sciences, 2011, 23(11): 1114-1129), a nanopore detection platform was constructed based on the patch clamp platform, and porin (Sigma-Aldrich, H9395-5mg) was inserted into the phospholipid bilayer membrane to form a single-channel nanopore.
2. The sequencing library obtained in Example 8 was added to the single-channel system, and the changes in current amplitude were detected and recorded through the patch clamp system.
3. A typical sequencing current diagram was shown in Fig. 12. The base identification software was used to convert the current signal into the read sequence SEQ ID NO.11, the SEQ ID NO.9 was used as the Subject sequence, the SEQ ID NO.11 was used as the Query sequence, and the BlastN algorithm was used for sequence alignment, and the results were shown in Fig. 13.

Fig. 13 showed that the sequence identity between the read sequence SEQ ID NO.11 and the target sequencing sequence was 83.9%, and the insertion & deletion (Gap) ratio was 8.4%. The identity and the insertion & deletion ratio were comparable to those of the commercial nanopore sequencing platform (Oxford Nanopore). However, the sequencing of this solution would not detect the adapter sequence, so it had significant advantages.

Read sequence (SEQ ID NO.11):

5'-GTTCTTGCTTACTCGCTTTTTTTTTGGAATTTTTTTTTTTGGAATTTTTTTT-
GCGG-3'

Although various embodiments of the present invention have been shown and described herein, it will be apparent to those skilled in the art that such embodiments are for illustrative purposes only. Various modifications, variations and substitutions will be apparent to those skilled in the art without departing from the present invention. Those skilled in the art will appreciate that various alternatives to the embodiments described herein may be adopted.

## Claims

1. A nucleic acid molecule capable of blocking a motor protein, wherein the nucleic acid molecule comprises at its end a modified nucleotide capable of blocking a motor protein, and the nucleic acid molecule is RNA or DNA.

2. The nucleic acid molecule according to claim 1, wherein the modified nucleotide comprises a group capable of blocking a motor protein; preferably, the group capable of blocking a motor protein is selected from: alkyl, fluorophore, streptavidin and/or biotin, cholesterol, methylene blue, dinitrophenol (DNP), digoxigenin ligand and/or anti-digoxigenin ligand and dibenzocyclooctynyl.

3. The nucleic acid molecule according to claim 1, wherein the α (alpha)-phosphate of the modified nucleotide located at the end of the nucleic acid molecule is modified; preferably, one negatively charged oxygen atom in the α-phosphate is substituted by the group capable of blocking a motor protein as described in claim 2; more preferably, one negatively charged oxygen atom in the α-phosphate is substituted by alkyl, most preferably, by methyl, ethyl, propyl, isopropyl, silylmethyl or boranyl.

4. The nucleic acid molecule according to claim 1, wherein the modified nucleotide is selected from the group consisting of: ribonucleotides or deoxyribonucleotides with alkyl substitution on the α-phosphate, optionally, wherein the alkyl is selected from methyl, ethyl, propyl, isopropyl, silylmethyl or boranyl; ribonucleotides or deoxyribonucleotides with nucleoside modification, for example, 3-methyladenine nucleotide, 7-methylguanine nucleotide, 1, N⁶-ethenoadenine nucleotide, hypoxanthine nucleotide, uracil nucleotide; and ribonucleotides or deoxyribonucleotides with modification at the sugar ring, for example, locked nucleotides, peptide nucleotides or threose nucleotides.

5. The nucleic acid molecule according to claim 3, wherein the modified nucleotide contained at the end of the nucleic acid molecule is represented by Formula I or Formula I': 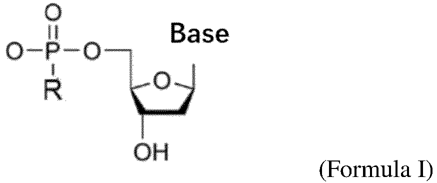 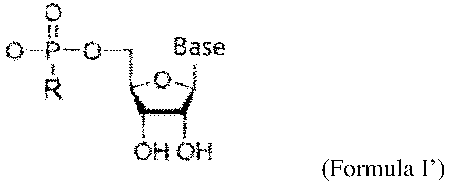
in Formula I or Formula I', Base represents any base,
R group is selected from the group consisting of: alkyl, fluorophore, streptavidin and/or biotin, cholesterol, methylene blue, dinitrophenol (DNP), digoxigenin ligand and/or anti-digoxigenin ligand and dibenzocyclooctynyl, preferably, the R group is alkyl, more preferably, the R group is methyl, ethyl, propyl, isopropyl, silylmethyl or boranyl; most preferably, the R group is methyl.

6. The nucleic acid molecule according to claim 5, wherein Base represents adenine (A) and R is methyl.

7. The nucleic acid molecule according to any one of claims 1 to 6, wherein the nucleic acid molecule comprises one modified nucleotide at the 5' end and/or one or more modified nucleotides at the 3' end.

8. A library, wherein the library comprises at least one nucleic acid molecule according to any one of claims 1 to 7.

9. A method for producing the nucleic acid molecule according to any one of claims 1 to 7, wherein the method comprises a step of making the end of the target nucleic acid molecule contain a modified nucleotide capable of blocking a motor protein.

10. The method according to claim 9, wherein the method comprises: introducing a phosphate group bearing a group capable of blocking a motor protein into the 5'-end nucleotide of the target nucleic acid molecule through 5'-end phosphorylation, preferably, the 5'-end phosphorylation is performed in the presence of a polynucleotide kinase (PNK).

11. The method according to claim 10, wherein a modified NTP represented by Formula IV is used as a reaction substrate: 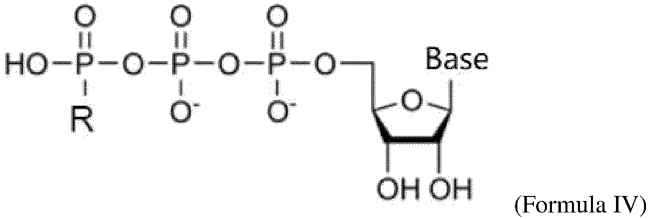
wherein, Base represents any base,
R group is selected from: methyl, ethyl, propyl, isopropyl, silylmethyl or boranyl, preferably methyl.

12. The method according to claim 11, wherein Base represents adenine (A), and R is methyl.

13. The method according to any one of claims 10 to 12, wherein the polynucleotide kinase (PNK) is selected from: nucleic acid kinase, phosphotransferase or phosphatase, preferably selected from: T4 phage-derived polynucleotide kinase, human-derived polynucleotide kinase 3'-phosphatase, human-derived polynucleotide 5'-hydroxykinase NOL9, or human-derived polynucleotide 5'-hydroxykinase Clp1.

14. The method according to claim 9, wherein the method comprises: introducing a modified nucleotide into the 3' end of the target nucleic acid molecule through a polymerase in a step of performing end-repair of the target nucleic acid molecule and/or a step of adding A to the 3' end of the target nucleic acid molecule.

15. The method according to claim 14, wherein a modified dNTP represented by Formula VI is used as a reaction substrate: 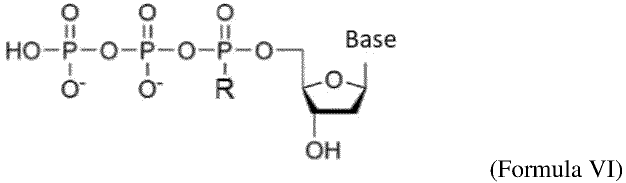
wherein, Base represents any base,
R group is selected from methyl, ethyl, propyl, isopropyl, silylmethyl or boranyl, preferably methyl.

16. The method according to claim 15, wherein Base represents adenine (A), and R is methyl.

17. The method according to claim 14, wherein one or more modified nucleotides are transferred to the 3' end of the target nucleic acid sequence.

18. The method according to any one of claims 14 to 17, wherein the polymerase is selected from nucleic acid polymerase, transcriptase, reverse transcriptase or terminal transferase, preferably selected from *Thermococcus gorgonarius* (Tgo) DNA polymerase or mutant thereof, *Thermus aquaticus* (Taq) polymerase I or *Escherichia coli* polymerase I Klenow fragment.

19. The nucleic acid molecule according to any one of claims 1 to 7 or the library according to claim 8, which is used in a single-molecular sequencing method, preferably, used in a nanopore sequencing method.

20. A single-molecule sequencing method, the method comprising:
(1) generating a sequencing nucleic acid molecule comprising at its end a modified nucleotide capable of blocking a motor protein or a library containing the sequencing nucleic acid molecule by the method according to any one of claims 9 to 18;
(2) ligating the sequencing nucleic acid molecule or library to a sequencing adapter and then incubating with a motor protein; alternatively, incubating a sequencing adapter with a motor protein and then ligating to the sequencing nucleic acid molecule or library;
(3) performing single-molecule sequencing on the product of step (2),
preferably, the single-molecule sequencing method is a nanopore sequencing method.

21. A method for detecting motor protein activity, the method comprising a step of incubating the nucleic acid molecule according to any one of claims 1 to 7 or the library according to claim 8 with a motor protein.
